(19) **Europäisches Patentamt**
**European Patent Office**
**Office européen des brevets**

(11) **EP 4 266 034 B1**

(12) **EUROPÄISCHE PATENTSCHRIFT**

(45) Veröffentlichungstag und Bekanntmachung des Hinweises auf die Patenterteilung:
**27.08.2025 Patentblatt 2025/35**

(51) Internationale Patentklassifikation (IPC):
**G01N 33/00** (2006.01)     **G01N 27/14** (2006.01)
**G01N 27/16** (2006.01)

(21) Anmeldenummer: **23168157.8**

(52) Gemeinsame Patentklassifikation (CPC):
**G01N 27/16; G01N 33/0047**

(22) Anmeldetag: **17.04.2023**

(54) **GASDETEKTIONSVORRICHTUNG UND GASDETEKTIONSVERFAHREN MIT EINEM SENSOR-BAUTEIL UND EINEM OXIDIERUNGS-BAUTEIL**

GAS DETECTION DEVICE AND GAS DETECTION METHOD USING A SENSOR COMPONENT AND AN OXIDATION COMPONENT

DISPOSITIF DE DÉTECTION DE GAZ ET PROCÉDÉ DE DÉTECTION DE GAZ UTILISANT UN COMPOSANT DE CAPTEUR ET UN COMPOSANT D'OXYDATION

(84) Benannte Vertragsstaaten:
**AL AT BE BG CH CY CZ DE DK EE ES FI FR GB GR HR HU IE IS IT LI LT LU LV MC ME MK MT NL NO PL PT RO RS SE SI SK SM TR**

(30) Priorität: **20.04.2022 DE 102022109534**
**01.07.2022 DE 102022116509**

(43) Veröffentlichungstag der Anmeldung:
**25.10.2023 Patentblatt 2023/43**

(73) Patentinhaber: **Dräger Safety AG & Co. KGaA**
**23560 Lübeck (DE)**

(72) Erfinder:
• **Baesler, Malte**
**23558 Lübeck (DE)**
• **Poethig, Tom**
**23558 Lübeck (DE)**

(74) Vertreter: **Meyer-Gramann, Klaus Dieter**
**c/o Drägerwerk AG & Co. KGaA**
**Moislinger Allee 53/55**
**23558 Lübeck (DE)**

(56) Entgegenhaltungen:
EP-A2- 0 157 247          DE-A1- 102008 028 682
DE-A1- 102021 116 050     JP-A- H09 218 176

**Beschreibung**

Die Erfindung betrifft eine Gasdetektionsvorrichtung und ein Verfahren, um einen räumlichen Bereich auf ein brennbares Zielgas zu überwachen.

Die DE 10 2008 028682 A1 offenbart Verfahren zum Betreiben eines in einem Messraum befindlichen Gassensors. Um auch kostengünstige Gassensoren in sensibler Umgebung mit einer größeren Funktionssicherheit und geringerem Aufwand betreiben zu können, wird der Gassensor in einem kleinen Messvolumen in wenigstens zwei Betriebsmodi abwechselnd diskontinuierlich betrieben. Durch katalytische Reinigung in einem der Betriebsmodi und/oder eine Herausfilterung sich nur über einen längeren Zeitraum hinweg ändernder Signalanteile kann dann eine basislinienunabhängige Vermessung erfolgen.

Es wird nicht offenbart, dass in einem Heiz-Zeitraum das Heizelement im eingeschalteten Zustand und das Oxidierungs-Bauteil in einem ausgeschalteten Zustand sind und im Oxidierungs-Zeitraum das Oxidierungs-Bauteil in einem eingeschalteten Zustand und das Heizelement in einem ausgeschalteten Zustand sind.

In einer Anwendung wird die Erfindung dafür verwendet, um das Vorhandensein eines brennbaren Zielgases, beispielsweise Methan ($CH_4$), auch dann zu detektieren, wenn die Konzentration des Zielgases gering ist, beispielsweise unter 10 ppm liegt.

Der Erfindung liegt die Aufgabe zugrunde, eine Gasdetektionsvorrichtung und ein Gasdetektionsverfahren bereitzustellen, die auch dann ein brennbares Zielgas mit einer hohen Zuverlässigkeit zu detektieren vermögen, wenn das Zielgas in einer relativ geringen Konzentration vorliegt und / oder variierende Umgebungsbedingungen einen relevanten Einfluss auf die Messung nehmen können.

Die Aufgabe wird durch eine Gasdetektionsvorrichtung mit den Merkmalen des Anspruchs 1 und durch ein Gasdetektionsverfahren mit den Merkmalen des Anspruchs 12 gelöst. Vorteilhafte Ausgestaltungen der erfindungsgemäßen Gasdetektionsvorrichtung sind, soweit sinnvoll, auch vorteilhafte Ausgestaltungen des erfindungsgemäßen Gasdetektionsverfahrens und umgekehrt.

Die erfindungsgemäße Gasdetektionsvorrichtung und das erfindungsgemäße Gasdetektionsverfahren vermögen einen räumlichen Bereich auf mindestens ein brennbares Zielgas zu überwachen, beispielsweise auf Methan ($CH_4$). Das erfindungsgemäße Verfahren wird unter Verwendung einer erfindungsgemäßen Gasdetektionsvorrichtung durchgeführt. Der räumliche Bereich ist beispielsweise ein Bereich einer Produktionsanlage oder eines Bergwerks das Innere eines Gebäudes oder Fahrzeugs oder Flugzeugs.

Im Folgenden ist von "dem brennbaren Zielgas" die Rede. Möglich ist, dass die Gasprobe in der Messkammer gleichzeitig mehrere brennbare Zielgase umfasst. Mit der Bezeichnung "das brennbare Zielgas" soll auch die Situation bezeichnet sein, dass verschiedene brennbare Zielgase in der Messkammer vorhanden sind. Der nachfolgend verwendete Begriff "Detektieren eines Zielgases" umfasst den Vorgang, das Vorhandensein mindestens eines Zielgases zu detektieren. In einer Ausgestaltung ist vorgegeben, welches brennbare Zielgas oder welche brennbaren Zielgase detektiert werden sollen. In einer anderen Anwendung soll jedes brennbare Zielgas im räumlichen Bereich detektiert werden.

Die Gasdetektionsvorrichtung umfasst eine Messkammer. Die Gasdetektionsvorrichtung ist so ausgestaltet und das Verfahren umfasst den Schritt, dass eine Gasprobe dauerhaft oder wenigstens zeitweise aus dem zu überwachenden Bereich in die Messkammer fließt, beispielsweise durch Ansaugen und / oder durch Diffundieren.

In oder an der Messkammer sind ein elektrisch leitendes Sensor-Bauteil sowie ein Oxidierungs-Bauteil angeordnet. Das Sensor-Bauteil weist eine messbare elektrische Detektionsgröße auf und kommt mit der Gasprobe in der Messkammer in einen flächigen Kontakt. Dieser Kontakt beeinflusst die messbare elektrische Detektionsgröße, insbesondere den elektrischen Widerstand, des Sensor-Bauteils wie folgt: In einer ersten Realisierungsform ist der Wert der Detektionsgröße des Sensor-Bauteils umso größer, je geringer die Konzentration des brennbaren Zielgases in der Gasprobe ist, welche sich in der Messkammer befindet. In einer zweiten Realisierungsform ist der Wert der Detektionsgröße umso kleiner, je geringer die Konzentration des brennbaren Zielgases in der Gasprobe ist, welche sich in der Messkammer befindet. In beiden Realisierungsformen korreliert die Detektionsgröße mit der Zielgas-Konzentration in der Gasprobe. Natürlich ist es möglich, dass die Gasprobe überhaupt kein Zielgas aufweist und daher die Detektionsgröße nicht in relevanter Weise beeinflusst oder im Vergleich zu einem Referenz-Zustand ohne Zielgas verändert wird.

Das Oxidierungs-Bauteil lässt sich für einen Einsatz einschalten und optional während des Einsatzes einschalten und ausschalten. Das eingeschaltete Oxidierungs-Bauteil vermag brennbares Gas in der Messkammer zu oxidieren - natürlich nur, wenn brennbares Gas in der Messkammer vorhanden ist. Anmerkung: Die weiter oben und nachfolgend verwendeten Begriffe "einschalten" und "ausschalten" können einen schlagartigen oder auch einen allmählichen Übergang von einem ausgeschalteten zu einem vollständig eingeschalteten Zustand bezeichnen.

Die Gasdetektionsvorrichtung umfasst weiterhin einen Detektions-Sensor. Der Detektions-Sensor vermag ein Maß für die Detektionsgröße des Sensor-Bauteils zu messen, beispielsweise ein Maß für den aktuellen elektrischen Widerstand oder für die elektrische Spannung, die am Sensor-Bauteil anliegt, oder für die Stärke des Stroms, der durch das Sensor-Bauteil fließt, oder für die gesamte elektrische Ladung.

Außerdem umfasst die Gasdetektionsvorrichtung eine signalverarbeitende Auswerteeinheit, die ein Signal vom Detektions-Sensor zu empfangen und zu verarbeiten vermag. In einer Ausgestaltung umfasst die Gasdetektionsvorrichtung ein Gehäuse, und die übrigen gerade erwähnten Bestandteile befinden sich im Inneren des Gehäuses. In einer anderen Ausgestaltung ist die Auswerteeinheit außerhalb des Gehäuses angeordnet, und das Signal des Detektions-Sensors wird an diese räumlich entfernte Auswerteeinheit übermittelt, bevorzugt drahtlos übermittelt.

Die Gasdetektionsvorrichtung ist dazu ausgestaltet, die folgenden Schritte durchzuführen, und das erfindungsgemäße Verfahren umfasst die folgenden Schritte:

- Das Oxidierungs-Bauteil wird oder ist eingeschaltet. In einem Oxidierungs-Zeitraum oxidiert das eingeschaltete Oxidierungs-Bauteil vollständig das oder jedes brennbare Zielgas in der Messkammer. Der Vorgang, dass das Oxidierungs-Bauteil brennbares Zielgas oxidiert, verringert die Menge von brennbarem Zielgas in der Messkammer und beseitigt optional, aber nicht notwendigerweise, alles brennbare Zielgas in der Messkammer. Selbstverständlich ist es möglich, dass bereits zu Beginn des Oxidierungs-Zeitraums kein brennbares Zielgas in der Messkammer vorhanden war, insbesondere dann, wenn der zu überwachende Bereich aktuell kein brennbares Zielgas aufweist.

- An einem Detektions-Zeitpunkt und an einem Referenz-Zeitpunkt misst der Detektions-Sensor die Detektionsgröße des Sensor-Bauteils - genauer: ein Maß für die Detektionsgröße zum jeweiligen Zeitpunkt. Der Detektions-Zeitpunkt liegt vor dem oder auch nach dem Referenz-Zeitpunkt. Dadurch, dass das Maß an zwei verschiedenen Zeitpunkten gemessen wird, liegen zwei Messwerte der Detektionsgröße vor (genauer gesagt: zwei Messwerte für das Maß für die Detektionsgröße), wobei die beiden Messwerte sich auf zwei unterschiedliche Zeitpunkte beziehen.

- Die Gasdetektionsvorrichtung wird so betrieben, dass folgende Situationen eintreten: Wenn im zu überwachenden Bereich brennbares Zielgas vorhanden ist, so ist am Detektions-Zeitpunkt auch in der Messkammer brennbares Zielgas vorhanden. Die Zielgas-Konzentration in der Messkammer kann genauso groß wie oder kleiner als die im überwachten Bereich sein. Am Referenz-Zeitpunkt ist dank des Oxidierens durch das Oxidierungs-Bauteil in der Messkammer weniger brennbares Zielgas vorhanden als am Detektions-Zeitpunkt oder sogar überhaupt kein brennbares Zielgas, auch wenn im Bereich brennbares Zielgas vorhanden ist. Falls im zu überwachenden Bereich brennbares Zielgas vorhanden ist, so ist dank des Oxidierens am Referenz-Zeitpunkt in der Messkammer eine geringere Konzentration von brennbarem Zielgas vorhanden als im Bereich und zum Detektions-Zeitpunkt in der Messkammer - oder sogar überhaupt kein brennbares Zielgas, selbst wenn im Bereich brennbares Zielgas vorhanden ist.

- Mindestens im Oxidierungs-Zeitraum ist das Oxidierungs-Bauteil eingeschaltet und vermag brennbares Zielgas in der Messkammer zu oxidieren. **In** einer ersten Alternative liegt der Detektions-Zeitpunkt am Beginn oder vor dem Beginn des Oxidierungs-Zeitraums, der Referenz-Zeitpunkt am Ende oder nach dem Ende des Oxidierungs-Zeitraums. **In** einer zweiten Alternative liegt umgekehrt der Referenz-Zeitpunkt am Beginn oder vor dem Beginn des Oxidierungs-Zeitraums, der Detektions-Zeitpunkt am Ende oder nach dem Ende des Oxidierungs-Zeitraums. Falls das Oxidierungs-Bauteil dauerhaft eingeschaltet ist, so wird der Zeitraum vom früheren zum späteren der beiden Zeitpunkte als der Oxidierungs-Zeitraum verwendet.

- Die Auswerteeinheit berechnet die Differenz zwischen dem Messwert für die Detektionsgröße des Sensor-Bauteils, den der Detektions-Sensor am Detektions-Zeitpunkt gemessen hat, und dem Messwert für die Detektionsgröße, den der Detektions-Sensor zum Referenz-Zeitpunkt gemessen hat. Am Detektions-Zeitpunkt ist in der Messkammer brennbares Zielgas vorhanden, falls im zu überwachenden Bereich brennbares Zielgas auftritt. Am Referenz-Zeitpunkt ist weniger oder sogar überhaupt kein brennbares Zielgas vorhanden.

- In einer ersten Alternative entscheidet die Auswerteeinheit automatisch abhängig von dieser Messwerte-Differenz, ob ein brennbares Zielgas in der Gasprobe vorhanden ist oder nicht. Diese Entscheidung hängt außerdem davon ab, ob die Detektionsgröße mit steigender Konzentration des Zielgases größer oder kleiner wird, und / oder vom Betrag der Differenz, also von $|\,\mathrm{dist}\,|$. In einer zweiten Alternative ermittelt die Auswerteeinheit automatisch abhängig von der Messwerte-Differenz wenigstens näherungsweise die Konzentration des brennbaren Zielgases in der Gasprobe. Bevorzugt wendet die Auswerteeinheit bei der zweiten Alternative einen abgespeicherten Zusammenhang zwischen der Messwerte-Differenz und der Zielgas-Konzentration auf die berechnete Differenz an. Mindestens bei der zweiten Alternative ist bevorzugt das Oxidierungs-Bauteil am Detektions-Zeitpunkt ausgeschaltet.

- Die beiden Alternativen, also das brennbare Zielgas detektiert und dessen Konzentration ermittelt wird, lassen sich miteinander kombinieren.

Gemäß der ersten Realisierungsform ist die Detektionsgröße des Sensor-Bauteils umso größer, desto geringer die Konzentration des Zielgases in der Messkammer ist. Daher ist der Messwert der Detektionsgröße zum Referenz-Zeitpunkt größer als der Messwert der Detektionsgröße zum Detektions-Zeitpunkt, vorausgesetzt in dem zu überwachenden Bereich und daher auch in der Messkammer ist ein brennbares Zielgas vorhanden. Gemäß der zweiten Realisierungsform ist entsprechend der Messwert der Detektionsgröße am Referenz-Zeitpunkt kleiner als der am Detektions-Zeitpunkt.

Falls im zu überwachenden Bereich und daher auch in der Messkammer kein brennbares Zielgas vorhanden ist, so sind die beiden Messwerte der Detektionsgröße, die an den beiden Zeitpunkten gemessen wurden, idealerweise gleich. In der Praxis kann die Differenz aufgrund unterschiedliche Umgebungsbedingungen auch dann ungleich Null sein, wenn im zu überwachenden Bereich kein brennbares Zielgas vorhanden ist und die Messkammer daher auch am Detektions-Zeitpunkt kein brennbares Zielgas aufweist. Diese Differenz zwischen den beiden Messwerten ist aber bei Abwesenheit von Zielgas in der Regel vom Betrag | dist | her geringer als die Differenz, die daraus resultiert, dass am Detektions-Zeitpunkt brennbares Zielgas in der Messkammer vorhanden ist und das Oxidierungs-Bauteil im Oxidierungs-Zeitraum wenigstens einen Teil dieses brennbaren Zielgases in der Messkammer oxidiert. Auch dann, wenn das Oxidierungs-Bauteil nur einen Teil oxidiert, aber nicht alles brennbare Zielgas in der Messkammer, ist in der Regel die Differenz größer als dann, wenn im Bereich und daher auch in der Messkammer kein brennbares Zielgas vorhanden ist. Daher ist eine Differenz ungleich Null oder außerhalb eines vorgegebenen Toleranzbands ein relativ sicheres Indiz dafür, dass in dem zu überwachenden Bereich und damit zum Detektions-Zeitpunkt auch in der Messkammer ein brennbares Zielgas vorhanden ist.

Die Detektionsgröße des Sensor-Bauteils ändert sich häufig in messbarer Weise durch den Kontakt mit brennbarem Zielgas, selbst wenn in der Messkammer brennbares Zielgas nur mit einer geringen Konzentration vorhanden ist. Jedoch hängt die Detektionsgröße in der Regel nicht nur von der Konzentration von brennbarem Zielgas, sondern auch von Umgebungsbedingungen ab, insbesondere von der Umgebungstemperatur und der Umgebungsfeuchte, optional auch des Umgebungsdrucks. Der Wert, den die Detektionsgröße bei einem Zustand annimmt, bei dem kein brennbares Zielgas in der Messkammer vorhanden ist, wird oft auch als Nullwert (Referenzwert) bezeichnet. Aus dem Stand der Technik bekannte Gasdetektionsvorrichtungen mit einem Sensor-Bauteil, das eine Detektionsgröße aufweist, die auf die Konzentration an brennbarem Zielgas reagiert, haben häufig folgende Nachteile: Der Nullpunkt hängt stark von Umgebungsbedingungen ab und ist in der Regel nicht bekannt. Oder ein Sensor für eine relevante Umgebungsbedingung wird benötigt, also ein zusätzlicher Sensor.

Die Erfindung löst dieses Problem dadurch, dass das Oxidierungs-Bauteil das brennbare Zielgas in der Messkammer im Oxidierungs-Zeitraum oxidiert und daher zum Referenz-Zeitpunkt quasi der aktuelle Nullpunkt für die Detektionsgröße des Sensor-Bauteils gemessen wird. Die Bezeichnung "aktueller Nullpunkt" deutet an, dass der Nullpunkt in der Regel von variierenden Umgebungsbedingungen abhängt und daher variieren kann. Die Zeitspanne zwischen den beiden Zeitpunkten ist in der Regel so kurz, dass sich die Umgebungsbedingungen in dieser Zeitspanne nicht wesentlich ändern und daher der Messwert zum Referenz-Zeitpunkt trotz des zeitlichen Abstands als Nullpunkt für die Messung zum Detektions-Zeitpunkt verwendet werden kann. Die erfindungsgemäß berechnete Differenz stimmt ausreichend genau mit der Differenz zwischen dem Messwert am Detektions-Zeitpunkt und dem (aktuellen) Nullpunkt am Detektions-Zeitpunkt überein.

Die Erfindung vermeidet die Notwendigkeit, die Gasdetektionsvorrichtung vor jedem Einsatz erneut justieren zu müssen, um einen aktuellen Nullpunkt aufzufinden. Wie gerade dargelegt, wird der Nullpunkt vielmehr wenigstens näherungsweise durch die Messung am Referenz-Zeitpunkt automatisch gewonnen. In vielen Fällen erspart die Erfindung auch die Notwendigkeit, dass die Gasdetektionsvorrichtung einen Sensor für eine Umgebungsbedingung umfassen muss.

Die Erfindung erspart auch die Notwendigkeit, die Gasdetektionsvorrichtung während des Einsatzes kalibrieren zu müssen, um die Gasdetektionsvorrichtung an eine mögliche Veränderung des Nullpunktes anzupassen. Bei Umgebungsbedingungen, die sich relativ rasch ändern, oder auch bei einer raschen Änderung der Zielgas-Konzentration kann das Ergebnis einer Kalibrierung, also eine Veränderung des Nullpunkts, bereits wieder veraltet sein, so dass die Gasdetektionsvorrichtung dann falsche Ergebnisse liefert. Die erfindungsgemäße Gasdetektionsvorrichtung führt hingegen in vielen Fällen auch bei sich rasch ändernden Umgebungsbedingungen und bei einer raschen Änderung der Zielgas-Konzentration zu zuverlässigen Ergebnissen, weil der Abstand zwischen den beiden Zeitpunkten ausreichend kurz gewählt werden kann und das Ergebnis der Messung und Auswertung nur von der Differenz abhängt, aber nicht von einem zuvor festgelegten Nullpunkt. Der Abstand zwischen den beiden Zeitpunkten lässt sich einerseits so groß wie nötig festlegen, nämlich so, dass zum Referenz-Zeitpunkt wenigstens ein Teil des brennbaren Zielgases oxidiert ist, und andererseits so klein wie möglich festlegen. Weil über das Vorhandensein von Zielgas anhand einer Differenz zwischen zwei aktuellen Messwerten entschieden wird, lässt sich in vielen Fällen auch Zielgas mit einer sehr kleinen Konzentration zuverlässig detektieren, und in vielen Fällen werden Fehlalarme vermieden. Dies wäre in vielen Fällen nur mit geringerer Zuverlässigkeit möglich, wenn die Detektion von der präzisen Kenntnis eines korrekten Nullpunkts abhänge.

Erfindungsgemäß weist das Sensor-Bauteil eine elektrische Detektionsgröße auf, die in einer ersten Realisierungsform umso größer und in einer zweiten Realisierungsform umso kleiner ist, je geringer die Zielgas-Konzentration ist. Diese Detektionsgröße ist insbesondere in einer Ausgestaltung der elektrische Widerstand, in einer anderen Ausgestaltung eine elektrische Kapazität und in einer dritten Ausgestaltung das elektrische Potenzial. Das Sensor-Bauteil umfasst beispielsweise

- einen elektrischen Halbleiter, dessen elektrischer Widerstand von der Zielgas-Konzentration abhängt, oder

- einen Wärmetönungssensor, dessen Temperatur von der Zielgas-Konzentration abhängt, oder
- einen photoelektrischen Sensor, der abhängig von der Intensität von auftreffender elektromagnetischer Strahlung ein elektrisches Signal erzeugt, wobei Zielgas diese elektromagnetische Strahlung dämpft,
- einen Photo-Ionisations-Detektor, der abhängig von einer Ionisierung ein elektrisches Signal erzeugt,
- einen photoakustischen Sensor, der abhängig von einem akustischen Effekt, der von der Zielgas-Konzentration abhängt, ein elektrisches Signal erzeugt, oder
- einen elektrochemischen Sensor, der einen elektrischen Strom erzeugt, dessen Stärke und / oder Spannung von der Zielgas-Konzentration abhängt.

Erfindungsgemäß oxidiert das Oxidierungs-Bauteil im Verlaufe des Oxidierungs-Zeitraums wenigstens einen Teil des brennbaren Zielgases in der Messkammer. Dieser oxidierte Teil ist bevorzugt wenigstens 30 %, besonders bevorzugt mindestens 50 %, insbesondere mindestens 80 % der Menge an brennbarem Zielgas, der zu Beginn des Oxidierungs-Zeitraums in der Messkammer vorhanden ist. In einer Ausgestaltung oxidiert das Oxidierungs-Bauteil im Oxidierungs-Zeitraum alles brennbare Zielgas in der Messkammer.

In einer Ausgestaltung umfasst die Gasdetektionsvorrichtung zusätzlich ein Heizelement. Dieses Heizelement vermag eine Gasprobe in der Messkammer zu heizen. Das Heizelement lässt sich einschalten und wieder ausschalten und lässt sich daher wahlweise in einem eingeschalteten und in einem ausgeschalteten Zustand betreiben. Gemäß dieser Ausgestaltung lässt sich auch das Oxidierungs-Bauteil einschalten und ausschalten und daher wahlweise in einem eingeschalteten oder in einem ausgeschalteten Zustand betreiben. Gemäß der Ausgestaltung mit dem Heizelement umfasst das Verfahren zusätzlich die folgenden Schritte, und die Gasdetektionsvorrichtung ist dazu ausgestaltet, die folgenden zusätzlichen Schritte durchzuführen:

- Zu Beginn eines Heiz-Zeitraums wird das Heizelement eingeschaltet. Am Ende des Heiz-Zeitraums wird das Heizelement wieder ausgeschaltet.
- Das eingeschaltete Heizelement erhitzt sich.
- Im Heiz-Zeitraum erhitzt das Heizelement im eingeschalteten Zustand das Gas in der Messkammer.
- Im Heiz-Zeitraum ist das Oxidierungs-Bauteil im ausgeschalteten Zustand. Umgekehrt ist das Heizelement wenigstens im Oxidierungs-Zeitraum im ausgeschalteten Zustand. Bevorzugt ist zu jedem Zeitpunkt entweder das Oxidierungs-Bauteil oder das Heizelement im jeweiligen eingeschalteten Zustand, aber niemals sind beide Bauteile gleichzeitig im jeweiligen eingeschalteten Zustand.

Erfindungsgemäß beeinflusst die gesuchte Zielgas-Konzentration die Detektionsgröße des Sensor-Bauteils. Die Detektionsgröße hängt in der Regel zusätzlich von der Temperatur des Sensor-Bauteils ab. Diese Temperatur wird vom eingeschalteten Oxidierungs-Bauteil sowie von dem Vorgang, das Oxidierungs-Bauteil einzuschalten und auszuschalten, beeinflusst. Die Ausgestaltung mit dem Heizelement reduziert den Einfluss, den das Oxidierungs-Bauteil auf das Sensor-Bauteil dadurch nimmt, dass es im eingeschalteten Zustand mehr Wärmeenergie einträgt als im ausgeschalteten Zustand. Indem der Einfluss reduziert wird, wird die Zuverlässigkeit des Detektionsergebnisses gesteigert. Idealerweise bewirken das eingeschaltete Heizelement und das eingeschaltete Oxidierungs-Bauteil den gleichen Eintrag von Wärmeenergie pro Zeiteinheit in oder auf das Sensor-Bauteil.

Gemäß der gerade beschriebenen Ausgestaltung ist das Heizelement im Heiz-Zeitraum eingeschaltet und im Oxidierungs-Zeitraum ausgeschaltet, bevorzugt während des gesamten Oxidierungs-Zeitraums. Das Oxidierungs-Bauteil ist im Oxidierungs-Zeitraum eingeschaltet und wenigstens in einem Abschnitt des Heiz-Zeitraums ausgeschaltet, bevorzugt im gesamten Heiz-Zeitraum ausgeschaltet. Bevorzugt überlappen sich also der Oxidierungs-Zeitraum und der Heiz-Zeitraum überhaupt nicht oder nur in einem Zeitpunkt. Möglich ist auch, dass in einem weiteren Zeitraum weder das Oxidierungs-Bauteil noch das Heizelement eingeschaltet sind.

In einer Realisierungsform fällt das Ende des Heiz-Zeitraums mit dem Beginn des Oxidierungs-Zeitraums zusammen. Oder das Ende des Oxidierungs-Zeitraums fällt mit dem Beginn des Heiz-Zeitraums zusammen. Diese beiden Ausgestaltungen reduzieren den Einfluss, den die Temperatur des Oxidierungs-Bauteils auf das Sensor-Bauteil nimmt, verglichen mit einer Ausgestaltung, bei der das Heizelement ausgeschaltet wird, dann eine Zeitspanne vergeht und erst dann das Oxidierungs-Bauteil eingeschaltet wird oder umgekehrt. Verglichen mit einer Ausgestaltung, bei der zeitweise sowohl das Heizelement als auch das Oxidierungs-Bauteil eingeschaltet sind, spart diese Realisierungsform elektrische Energie ein. Außerdem wird erleichtert, den Eintrag von Wärmeenergie pro Zeiteinheit in das Sensor-Bauteil konstant zu halten.

Bevorzugt befindet sich die Gasdetektionsvorrichtung während eines Einsatzes stets in genau einem der folgenden Zustände:

- Das Oxidierungs-Bauteil ist eingeschaltet, und das Heizelement ist ausgeschaltet.
- Das Heizelement ist eingeschaltet, und das Oxidierungs-Bauteil ist ausgeschaltet.

- Die Messkammer wird gespült, oder eine Gasprobe fließt in die Messkammer. Bevorzugt sind dann sowohl das Heizelement als auch das Oxidierungs-Bauteil ausgeschaltet.

Außerhalb eines Einsatzes kann die Gasdetektionsvorrichtung ausgeschaltet sein, sich also in einem Ruhezustand befinden.

Wie bereits dargelegt, bewirken sowohl das eingeschaltete Oxidierungs-Bauteil als auch das eingeschaltete Heizelement, dass Wärmeenergie auf das Sensor-Bauteil einwirkt. Bevorzugt ist der Eintrag von Wärmeenergie pro Zeiteinheit, den das eingeschaltete Heizelement bewirkt, genauso groß wie der Eintrag von Wärmeenergie pro Zeiteinheit durch das eingeschaltete Oxidierungs-Bauteil. Diese Aussage gilt für einen Zustand, bei dem sich kein brennbares Zielgas in der Messkammer befindet.

Der Eintrag von Wärmeenergie pro Zeiteinheit, den das Oxidierungs-Bauteil bewirkt, hängt von der Geometrie, insbesondere von der Oberfläche, und der Temperatur des Oxidierungs-Bauteils sowie von der Entfernung zwischen dem Oxidierungs-Bauteil und dem Sensor-Bauteil ab. Falls der Eintrag von Wärmeenergie pro Zeiteinheit gleich ist und in der Messkammer kein brennbares Zielgas vorhanden ist, so ist idealerweise auch der Wert der Detektionsgröße gleich, genauer gesagt: Bei eingeschaltetem Oxidierungs-Bauteil und ausgeschaltetem Heizelement nimmt die Detektionsgröße idealerweise den gleichen Wert an wie bei ausgeschaltetem Oxidierungs-Bauteil und eingeschaltetem Heizelement. Dies gilt optional erst nach einer Einschwingphase, die auftritt, wenn das Oxidierungs-Bauteil oder das Heizelement eingeschaltet wird oder ausgeschaltet wird.

Die gerade beschriebene Realisierungsformen des Heizelements erspart in vielen Fällen die Notwendigkeit, die Temperatur des Detektions-Sensors, des Oxidierungs-Bauteils oder des Heizelements zu regeln, insbesondere auf einen konstanten Wert zu regeln.

Erfindungsgemäß ist das Oxidierungs-Bauteil wenigstens im Oxidierungs-Zeitraum eingeschaltet. **In** einer Ausgestaltung ist das Oxidierungs-Bauteil mindestens in einem Einlass-Zeitraum ausgeschaltet. Das Oxidierungs-Bauteil wird im laufenden Betrieb also eingeschaltet und / oder ausgeschaltet.

Mindestens im Einlass-Zeitraum ist eine Fluidverbindung zwischen der Messkammer und der Umgebung hergestellt, und die Gasprobe fließt aus dem zu überwachenden räumlichen Bereich in die Messkammer, beispielsweise durch Diffusion oder Ansaugen. Dieser Einlass-Zeitraum liegt vor dem Oxidierungs-Zeitraum und überlappt idealerweise nicht oder nur in einem Zeitpunkt mit dem Oxidierungs-Zeitraum. Der Detektions-Zeitpunkt liegt außerhalb des Oxidierungs-Zeitraums, bevorzugt im Einlass-Zeitraum, im Einlass-Zeitraum, besonders bevorzugt am Anfang des Einlass-Zeitraums. Weil im Einlass-Zeitraum das Oxidierungs-Bauteil durchgehend oder zumindest überwiegend ausgeschaltet ist, oxidiert es im Einlass-Zeitraum kein oder nur vernachlässigbar wenig brennbares Zielgas. Daher sammelt sich im Einlass-Zeitraum brennbares Zielgas in der Messkammer an, vorausgesetzt der zu überwachende Bereich enthält brennbares Zielgas.

Die Ausgestaltung mit dem Oxidierungs-Bauteil, das im laufenden Betrieb eingeschaltet und oder ausgeschaltet wird, ermöglicht es in vielen Fällen, dass die Messkammer dauerhaft mit dem zu überwachenden Bereich in einer Fluidverbindung steht. Nicht erforderlich ist, einen Verschluss für einen Einlass zur Messkammer im laufenden Betrieb zu öffnen und zu verschließen. Insbesondere bei einer relativ geringen Konzentration von brennbarem Zielgas oxidiert das eingeschaltete Oxidierungs-Bauteil sehr rasch brennbares Zielgas in der Messkammer, sodass die Fluidverbindung nicht zu einer nennenswerten Verfälschung von Messergebnissen steht.

**In** einer Ausgestaltung liegt der Detektions-Zeitpunkt am Anfang des Oxidierungs-Zeitraums. **In** einer bevorzugten Ausgestaltung tritt zwischen dem Einlass-Zeitraum und dem Oxidierungs-Zeitraum ein zeitlicher Abstand auf. **In** diesem zeitlichen Abstand liegt ein Heiz-Zeitraum. Bevorzugt wird das optionale Heizelement am Ende des Einlass-Zeitraums eingeschaltet und am Ende des Einlass-Zeitraums, also vor oder zu Beginn des Oxidierungs-Zeitraumes, wieder ausgeschaltet.

Erfindungsgemäß ist das Oxidierungs-Bauteil wenigstens im Verlaufe des Oxidierungs-Zeitraums eingeschaltet und oxidiert brennbares Zielgas in der Messkammer. Die nachfolgend beschriebene Ausgestaltung lässt sich mit einem Oxidierungs-Bauteil kombinieren, das sich einschalten und ausschalten lässt. Die nachfolgend beschriebene Ausgestaltung erspart aber die Notwendigkeit, das Oxidierungs-Bauteil im laufenden Betrieb einzuschalten und / oder auszuschalten. Das Oxidierungs-Bauteil kann im laufenden Einsatz auch dauerhaft eingeschaltet sein. Gemäß dieser anderen Ausgestaltung lässt sich die Messkammer wahlweise in einem geöffneten Zustand oder in einem geschlossenen Zustand betreiben. Wenn die Messkammer im geöffneten Zustand ist, so kann die Gasprobe aus dem zu überwachenden Bereich in die Messkammer fließen. Im geöffneten Zustand ist also eine Fluidverbindung zwischen der Messkammer und dem zu überwachenden Bereich hergestellt. Im geschlossenen Zustand ist die Messkammer gegen den Bereich fluiddicht abgedichtet. "Fluiddicht" bedeutet: bis auf unvermeidliche Spalten oder Lücken. Bevorzugt ist in einem Ruhezustand die Gasdetektionsvorrichtung ausgeschaltet und fluiddicht von der Umgebung getrennt.

In dieser Ausgestaltung umfasst die Gasdetektionsvorrichtung eine verschließbare Öffnung, beispielsweise ein Ventil oder eine Aussparung mit einer Klappe oder eine Blende für die Öffnung. Wenn diese Öffnung geöffnet ist, kann eine Gasprobe aus der Umgebung in die Messkammer fließen. Wenn die Öffnung geschlossen ist, ist die Messkammer gegen die Umgebung abgedichtet, sodass dann keine Gasprobe in die Messkammer fließen kann. Bevorzugt ist die Öffnung im Oxidierungs-Zeitraum geschlossen und wenigstens zeitweise vor und / oder nach dem Oxidierungs-Zeitraum geöffnet. Möglich ist aber auch, dass auch im Oxidierungs-Zeitraum Gas in die Messkammer fließen kann. In der Regel ist die Menge des Zielgases, welches bei geöffnetem Verschluss im Oxidierungs-Zeitraum in die Messkammer fließt, geringer als die Menge, welche im Oxidierungs-Zeitraum vom Oxidierungs-Bauteil oxidiert wird.

In einem Einlass-Zeitraum ist die Messkammer im geöffneten Zustand. Bevorzugt umfasst dieser Einlass-Zeitraum den Detektions-Zeitpunkt, oder der Einlass-Zeitraum liegt vor dem Detektions-Zeitpunkt. Am Referenz-Zeitpunkt ist die Messkammer bevorzugt im geschlossenen Zustand.

Mindestens im Oxidierungs-Zeitraum ist die Messkammer im geschlossenen Zustand, und das Oxidierungs-Bauteil oxidiert brennbares Zielgas in der Messkammer, idealerweise alles brennbaren Zielgas. Weil die Messkammer im geschlossenen Zustand ist, kann kein brennbares Zielgas aus dem Bereich in die Messkammer fließen. In dem bereits erwähnten Einlass-Zeitraum ist die Messkammer im geöffneten Zustand. In diesem Einlass-Zeitraum sammelt sich brennbares Zielgas in der Messkammer an, vorausgesetzt im zu überwachenden Bereich ist brennbares Zielgas vorhanden.

Diese beiden Ausgestaltungen lassen sich kombinieren, beispielsweise wie folgt: Im Oxidierungs-Zeitraum ist das Oxidierungs-Bauteil eingeschaltet, und die Messkammer ist im geschlossenen Zustand. Im Einlass-Zeitraum ist das Oxidierungs-Bauteil ausgeschaltet, und die Messkammer ist im geöffneten Zustand. Diese Kombination der beiden Ausgestaltungen ermöglicht es in vielen Fällen, mit noch größerer Zuverlässigkeit das Zielgas zu entdecken, auch wenn es nur in einer relativ geringen Konzentration in dem zu überwachenden Bereich vorhanden ist. Optional befindet sich zwischen dem Einlass-Zeitraum und dem Oxidierungs-Zeitraum ein Heiz-Zeitraum.

In einer ersten Alternative der Erfindung liegt der Detektions-Zeitpunkt vor dem Referenz-Zeitpunkt. Eine Gasprobe ist spätestens bis zum Detektions-Zeitpunkt in die Messkammer geflossen. Der Oxidations-Zeitraum beginnt am oder nach dem Detektions-Zeitpunkt und endet vor dem oder am Referenz-Zeitpunkt. Bevorzugt wird zwischen dem Detektions-Zeitpunkt und dem Referenz-Zeitpunkt das Oxidations-Bauteil eingeschaltet, und / oder die Messkammer wird fluiddicht vom zu überwachenden Bereich getrennt.

In der zweiten Alternative der Erfindung liegt umgekehrt der Referenz-Zeitpunkt vor dem Detektions-Zeitpunkt. Der Oxidations-Zeitraum beginnt am oder nach dem Referenz-Zeitpunkt und endet vor dem oder am Detektions-Zeitpunkt. In einer Ausgestaltung ist die Messkammer wenigstens im Oxidations-Zeitraum fluiddicht gegen den Bereich abgetrennt. Bis zum Referenz-Zeitpunkt hat das Oxidierungs-Bauteil brennbares Zielgas in der Messkammer oxidiert. Nach dem Referenz-Zeitpunkt und mindestens bis zum Detektions-Zeitpunkt, optional auch noch danach, fließt die Gasprobe in die Messkammer. Bevorzugt wird zwischen dem Referenz-Zeitpunkt und dem Detektions-Zeitpunkt das Oxidations-Bauteil ausgeschaltet, und / oder eine Fluidverbindung zwischen der Messkammer und den Bereich wird hergestellt.

Möglich ist, dass die Gasdetektionsvorrichtung so betrieben wird, dass nacheinander mehrere Oxidations-Zeiträume auftreten, wobei zwischen zwei aufeinanderfolgenden Oxidations-Zeiträumen eine Lücke auftritt und das Oxidierungs-Bauteil mindestens in jedem Oxidierungs-Zeitraum Zielgas in der Messkammer oxidiert. Für jeden Oxidierungs-Zeitraum wird das Maß für die Detektionsgröße an jeweils einem Detektions-Zeitpunkt und an einem Referenz-Zeitpunkt gemessen, und die erfindungsgemäße Auswertung wird für jeden Oxidierungs-Zeitraum erneut durchgeführt. Dadurch lässt sich in vielen Fällen brennbares Zielgas relativ rasch detektieren.

Bei den nachfolgend beschriebenen Ausgestaltungen lässt sich bevorzugt das Oxidierungs-Bauteil einschalten und ausschalten. In einer ersten Ausgestaltung ist eine Oxidierungs-Zeitspanne fest vorgegeben. Die Dauer des oder jedes Oxidierungs-Zeitraums ist gleich dieser Oxidierungs-Zeitspanne. Die Oxidierungs-Zeitspanne und damit jeder Oxidierungs-Zeitraum sind einerseits so lang wie nötig und andererseits so kurz wie möglich. "So lang wie nötig" bedeutet: Auch bei der höchsten zu erwartenden Konzentration von brennbarem Zielgas in der Messkammer oxidiert das Oxidierungs-Bauteil im Oxidierungs-Zeitraum das brennbare Zielgas in der Messkammer, sodass am Ende des Oxidierungs-Zeitraums die Messkammer frei von brennbarem Zielgas ist.

In einer zweiten Ausgestaltung hängt die Dauer des oder mindestens eines Oxidierungs-Zeitraums, also wie lange das Oxidierungs-Bauteil eingeschaltet ist, von der Konzentration von brennbarem Zielgas in der Messkammer ab. Die zweite Ausgestaltung erspart die Notwendigkeit, eine Oxidierungs-Zeitspanne fest vorzugeben. Gemäß der zweiten Ausgestaltung ist die Gasdetektionsvorrichtung zusätzlich dazu ausgestaltet, die folgenden Schritte durchzuführen, und das Verfahren umfasst zusätzlich die folgenden Schritte:

Mindestens einmal wird eine Steigungs-Berechnungs-Abfolge durchgeführt. Die oder jede Steigungs-Berechnungs-Abfolge umfasst die folgenden Schritte:

- Das Maß für die Detektionsgröße wird an mindestens zwei Zeitpunkten gemessen, wobei diese Zeitpunkte zeitlich voneinander beabstandet sind. Beide Zeitpunkte liegen im Oxidierungs-Zeitraum. Bevorzugt wird das Maß für die Detektionsgröße am Detektions-Zeitpunkt und an mindestens einen weiteren Zeitpunkt, der zeitlich nach dem Detektions-Zeitpunkt liegt, gemessen. Bevorzugt wird eine Abtastrate für die Messung der Detektionsgröße vorgegeben, und diese Abtastrate legt den zeitlichen Abstand zwischen zwei unmittelbar aufeinanderfolgenden Mess-Zeitpunkten fest.

- Durch die Messungen wird näherungsweise der zeitliche Verlauf der Detektionsgröße während des Oxidierens ermittelt. Falls in der Messkammer brennbares Zielgas vorhanden ist, so nimmt die Detektionsgröße gemäß der ersten Realisierungsform des Sensor-Bauteils mit der Zeit zu, bis alles Zielgas oxidiert ist. Die Steigung des zeitlichen Verlaufs der Detektionsgröße ist also positiv, bis alles Zielgas oxidiert ist. Gemäß der zweiten Realisierungsform nimmt die Detektionsgröße ab, und die Steigung ist negativ, bis alles Zielgas oxidiert ist. Falls in der Messkammer kein brennbares Zielgas vorhanden ist, so bleibt die Detektionsgröße bei beiden Realisierungsformen im Oxidierungs-Zeitraum annähernd konstant.

- Abhängig von den gemessenen Werten der Detektionsgröße wird ein Maß für die Steigung der Detektionsgröße, also für die Ableitung des zeitlichen Verlaufs der Detektionsgröße nach der Zeit, berechnet, bevorzugt von der Auswerteeinheit. Im einfachsten Fall ist dieses Maß für die Steigung die Differenz zwischen den beiden Messwerten der Detektionsgröße, die an den zeitlich letzten beiden Zeitpunkten gemessen wurden. Die berechnete Steigung kann zeitlich veränderlich sein.

Falls die Steigung des zeitlichen Verlaufs der Detektionsgröße vom Betrag her unter einer vorgegebenen Schranke liegt, wird die Messung der Detektionsgröße beendet. Der zeitlich letzte Zeitpunkt wird als der Referenz-Zeitpunkt verwendet. Der Messwert am zeitlich letzten Zeitpunkt wird als der Messwert am Referenz-Zeitpunkt verwendet. Die Schranke kann gleich Null oder größer als Null sein. Falls die Steigung unterhalb der vorgegebenen Schranke liegt, ist praktisch alles Zielgas in der Messkammer oxidiert.

Bei der zweiten Ausgestaltung ist in der Regel der Oxidierungs-Zeitraum umso länger, je höher die Konzentration von brennbarem Zielgas in der Messkammer ist. Die zweite Ausgestaltung führt in vielen Fällen dann zu einem besonders raschen Detektionsergebnis, wenn in dem zu überwachenden Bereich und daher in der Messkammer kein brennbares Zielgas vorhanden ist. In diesem Falle wird die Steigung nur von Umgebungsbedingungen beeinflusst und bleibt in der Regel unter der vorgegebenen Schranke. Andererseits führt die zweite Ausgestaltung auch dann zu einem zuverlässigen Messergebnis, wenn eine sehr hohe Konzentration von brennbaren Zielgas vorhanden ist. In der Regel wird auch in diesem Fall alles brennbare Zielgas in der Messkammer oxidiert, sodass auch in diesem Fall der Messwert am zweiten Zeitpunkt (am zeitlich letzten Zeitpunkt) zuverlässig als Nullwert fungiert.

In einer dritten Ausgestaltung ist ein funktionaler Zusammenhang vorgegeben, der den zeitlichen Verlauf der Detektionsgröße während des Oxidierungs-Zeitraums beschreibt. Dieser funktionale Zusammenhang umfasst mindestens einen Modellparameter. Häufig hat dieser funktionale Zusammenhang die Gestalt einer Exponentialfunktion, also

$$f(t) = A - C*exp(-\alpha*t) \text{ oder } f(t) = A*[1- C*exp(-\alpha*t)]$$

mit den Modellparametern A, C und $\alpha$.

Während des Oxidierungs-Zeitraums wird die Detektionsgröße (genauer gesagt: das Maß für die Detektionsgröße) mehrmals gemessen, wodurch eine Stichprobe gewonnen wird. Mithilfe dieser Stichprobe werden automatisch Werte für die Modellparameter berechnet. Durch eine Extrapolation lässt sich häufig mit ausreichender Zuverlässigkeit der Messwert für den zweiten Zeitpunkt vorhersagen. Die dritte Ausgestaltung führt häufig sowohl bei einer niedrigen als auch bei einer höheren Zielgas-Konzentration rasch zu einem zuverlässigen Messergebnis.

Auch bei der dritten Ausgestaltung braucht keine feste Oxidations-Zeitspanne vorgegeben zu werden. Möglich ist aber auch, eine feste Anzahl N > 1 von Messwerten für die Stichprobe, also für die Anzahl der Stichprobenelemente, vorzugeben und den Oxidations-Zeitraum zu beenden, wenn die N Messwerte für die Stichprobe vorliegen.

Die zweite Ausgestaltung und die dritte Ausgestaltung lassen sich miteinander kombinieren.

In einer Realisierungsform umfasst das Oxidierungs-Bauteil das elektrisch leitende Sensor-Bauteil. Falls das Oxidierungs-Bauteil eingeschaltet ist, so fließt elektrischer Strom durch das Sensor-Bauteil. Bei ausgeschaltetem Oxidierungs-Bauteil fließt kein elektrischer Strom durch das Sensor-Bauteil. Beispielsweise ist das Oxidierungs-Bauteil als ein sogenannter Pellistor ausgestaltet und umfasst ein Heizelement, welches als das Sensor-Bauteil fungiert. Das Oxidierungs-Bauteil umfasst weiterhin eine keramische Ummantelung um das Heizelement und eine katalytische Beschichtung auf oder eine katalytische Beimischung in der keramischen Ummantelung. Das Heizelement fungiert als das Sensor-Bauteil. Der Detektions-Sensor misst eine Detektionsgröße des Heizelements.

In einer anderen Realisierungsform sind das Oxidierungs-Bauteil und das Sensor-Bauteil voneinander getrennt. An das Sensor-Bauteil lässt sich eine elektrische Spannung anlegen, und als Folge hiervon fließt elektrischer Strom durch das Sensor-Bauteil, und zwar unabhängig davon, ob das Oxidierungs-Bauteil eingeschaltet oder ausgeschaltet ist. Gemäß dieser anderen Realisierungsform wird das Oxidierungs-Bauteil lediglich dafür benutzt, möglicherweise vorhandenes brennbares Zielgas in der Messkammer zu oxidieren, aber nicht dafür, dieses Zielgas auch zu detektieren. Der Detektions-Sensor misst bevorzugt nicht eine Größe des Oxidierungs-Bauteils. Auch bei dieser anderen Realisierungsform kann das Oxidierungs-Bauteil als ein Pellistor ausgestaltet sein.

In einer Anwendung grenzt der zu überwachende räumliche Bereich direkt an die Gasdetektionsvorrichtung an, und die Gasprobe kann durch einen Einlass in die Messkammer fließen. In einer anderen Anwendung tritt zwischen dem zu überwachenden räumlichen Bereich und der Gasdetektionsvorrichtung ein Abstand auf. Die Gasprobe kann nur durch eine Fluidführungseinheit hindurch aus dem räumlichen Bereich und einem Einlass hindurch zur Messkammer fließen, aber nicht an dieser Fluidführungseinheit vorbei. Dank des räumlichen Abstands ist die Gasdetektionsvorrichtung vor Umgebungseinflüssen in dem zu überwachenden Bereich weitgehend geschützt. Die Fluidführungseinheit kann insbesondere die Form eines Schlauchs oder eine Röhre aufweisen. Bevorzugt saugt die Gasdetektionsvorrichtung eine Gasprobe aus dem zu überwachenden Bereich durch die Fluidführungseinheit hindurch an.

In einer Ausgestaltung ist die Fluidfördereinheit fluiddicht mit einem Adapter verbunden. Der Adapter lässt sich auf die Gasdetektionsvorrichtung aufsetzen und wieder von der Gasdetektionsvorrichtung abnehmen. Bei aufgesetztem Adapter kann die Gasprobe nur durch die Fluidführungseinheit hindurch zur Messkammer fließen, bei abgenommenem Adapter direkt aus dem räumlichen Bereich zur Messkammer.

Die erfindungsgemäße Gasdetektionsvorrichtung kann als ein tragbares Gerät ausgestaltet sein, wobei ein Benutzer dieses tragbare Gerät mit sich führt. Bevorzugt umfasst dieses tragbare Gerät eine eigene Spannungsversorgungseinheit. Die erfindungsgemäße Gasdetektionsvorrichtung kann auch als stationäres Gerät ausgestaltet sein und wenigstens zeitweise mit einem stationären Spannungsversorgungsnetz verbunden sein. Die erfindungsgemäße Gasdetektionsvorrichtung kann eine Ausgabeeinheit umfassen, wobei auf diese Ausgabeeinheit ein Alarm oder die ermittelte Konzentration ausgegeben werden.

Im Folgenden wird die Erfindung anhand eines Ausführungsbeispiels beschrieben. Hierbei zeigt

Figur 1     schematisch eine erste Ausführungsform der erfindungsgemäßen Gasdetektionsvorrichtung;

Figur 2     schematisch eine zweite Ausführungsform der erfindungsgemäßen Gasdetektionsvorrichtung;

Figur 3     beispielhaft eine Abfolge mit Messungen der Gasdetektionsvorrichtung;

Figur 4     beispielhaft das Oxidierungs-Bauteil;

Figur 5     ein beispielhaftes Flussdiagramm für den Einsatz der Gasdetektionsvorrichtung.

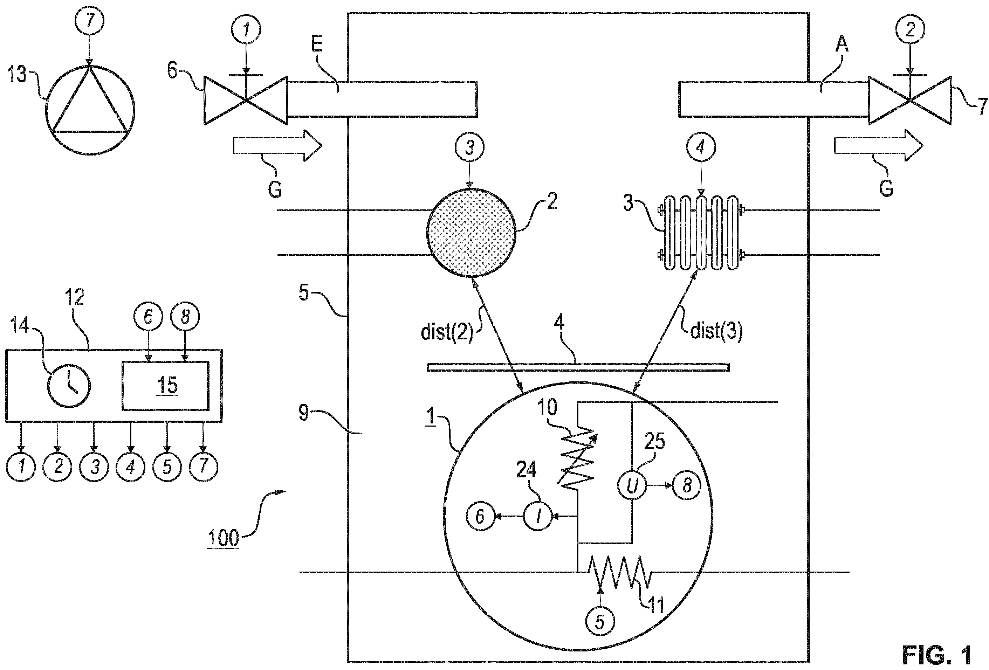

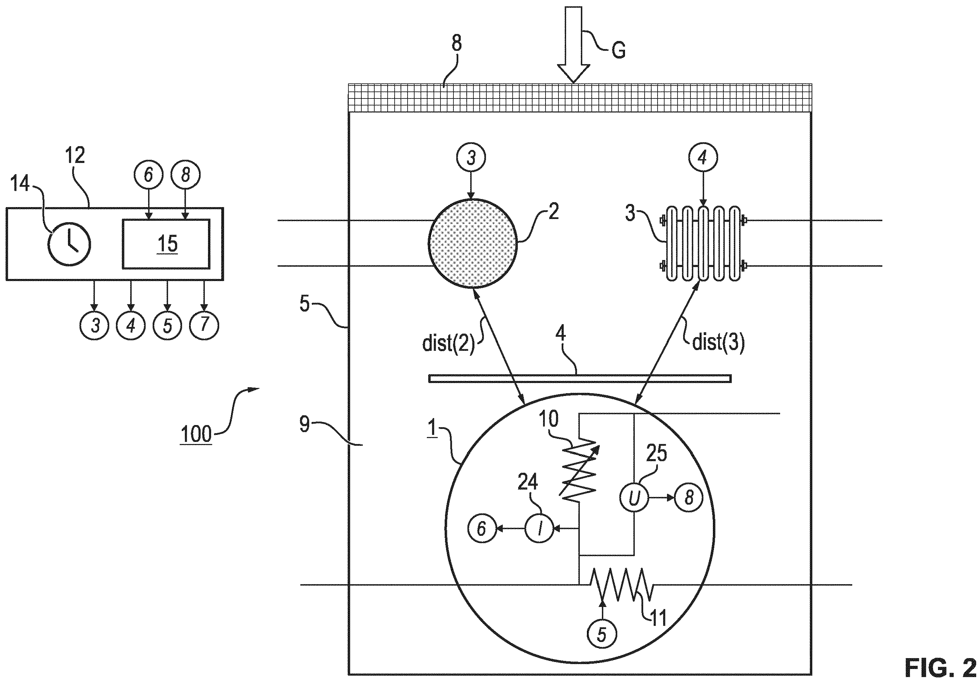

Figur 1 zeigt schematisch eine erste Ausführungsform der erfindungsgemäßen Gasdetektionsvorrichtung 100, Figur 2 eine zweite Ausführungsform.

Die Gasdetektionsvorrichtung 100 wird in einer Anwendung dafür verwendet, um einen räumlichen Bereich auf das Vorhandensein eines brennbaren Gases zu überwachen und / oder um die Konzentration eines brennbaren Gases zu ermitteln. Dieses brennbare Gas wird im Folgenden als "Zielgas" bezeichnet. Der räumliche Bereich ist beispielsweise eine Raffinerie oder eine sonstige Produktionsanlage, das Innere eines Gebäudes, ein Bergwerk, ein Fahrzeug oder ein Flugzeug. Das brennbare Gas ist beispielsweise Methan ($CH_4$).

In einer anderen Anwendung wird die Gasdetektionsvorrichtung 100 dafür verwendet, um einen Probanden auf Alkohol zu untersuchen. Bekanntlich enthält die Luft, die ein Proband ausatmet, Atemalkohol, falls dieser Proband Alkohol zu sich genommen hat und daher in seinem Blut und / oder in seinem Mund noch Alkohol vorhanden ist. Bei dieser Anwendung umfasst die Gasdetektionsvorrichtung 100 ein Mundstück. Der Proband atmet in das Mundstück hinein, und wenigstens ein Teil der abgegebenen Atemprobe gelangt in das Innere der Gasdetektionsvorrichtung 100. Bei dieser Anwendung ist also gasförmiger Atemalkohol das brennbare Zielgas.

Die Gasdetektionsvorrichtung 100 ist in einer Realisierungsform ein tragbares Gerät, welches ein Mensch in einer Hand halten oder auch an seiner Kleidung oder Schutzausrüstung befestigen kann und welches eine eigene Spannungsversorgungseinheit umfasst. Beispielsweise führt ein Benutzer eine solche Gasdetektionsvorrichtung 100 mit sich, während er sich in einem Bereich aufhält, in dem mindestens ein brennbares Zielgas vorhanden sein kann. Oder ein Proband nimmt die Gasdetektionsvorrichtung 100 in eine Hand und gibt eine Atemprobe in das Mundstück ein. Die Gasdetektionsvorrichtung 100 kann auch als ein stationäres Gerät ausgestaltet sein, welches sich an ein stationäres Spannungsversorgungsnetz anschließen lässt und nicht notwendigerweise eine eigene Spannungsversorgungseinheit aufweist.

Die Gasdetektionsvorrichtung 100 umfasst ein Gehäuse 5, welches eine Messkammer 9 - bis auf nachfolgend beschriebene Öffnungen und bis auf unvermeidliche Spalten und Schlitze - fluiddicht umgibt.

Bei der ersten Ausführungsform führt ein Einlass E im Gehäuse 5 von der Umgebung in die Messkammer 9, und ein Auslass A im Gehäuse 5 führt aus der Messkammer 9 in die Umgebung. Ein ansteuerbares Ventil 6 vermag wahlweise den Einlass E freizugeben oder zu versperren. Ein ansteuerbares Ventil 7 vermag wahlweise den Auslass A freizugeben oder zu versperren. Bei geöffnetem Ventil 6 kann eine Gasprobe G aus der Umgebung, also aus dem zu überwachenden Bereich, durch den Einlass E hindurch in die Messkammer 9 fließen. Bei geöffnetem Ventil 7 kann die Gasprobe G aus der Messkammer 9 heraus in die Umgebung fließen. Ein signalverarbeitendes Steuergerät (control unit) 12 mit einer Systemuhr 14 vermag automatisch die Ventile 6 und 7 sowie weitere nachfolgend beschriebene Bauteile anzusteuern.

Optional vermag das Steuergerät 12 eine Pumpe 13 anzusteuern, und die angesteuerte und dadurch aktivierte Pumpe 13 saugt die Gasprobe G durch den geöffneten Einlass E hindurch in das Innere der Gasdetektionsvorrichtung 100. Bei geöffnetem Auslass A fließt gleichzeitig Gas aus der Messkammer 9 heraus. Möglich ist auch, dass die Gasprobe G durch den Einlass E hindurch in das Innere der Gasdetektionsvorrichtung 100 hinein diffundiert.

Möglich ist auch eine Realisierungsform, bei der zwar die Pumpe 13, der Einlass E und der Auslass A vorhanden sind, aber nicht die Ventile 6 und 7. Möglich ist, dass der Einlass E und der Auslass A dauerhaft geöffnet sind. Möglich ist auch, dass das Ventil 6 durch eine andere Form eines Verschlusses ersetzt ist, beispielsweise durch eine Lochblende mit einem Lochmuster, wobei das Lochmuster mindestens ein Loch aufweist und wobei die Lochblende relativ zum Gehäuse 5 beweglich ist. Je nach Stellung der Lochblende relativ zum Gehäuse 5 überlappt das oder ein Loch der Lochmuster mit dem Einlass **E,** und der Einlass E ist geöffnet, oder die Lochblende verschließt den Einlass E.

Die Pumpe 13 kann während des Betriebs der Gasdetektionsvorrichtung 100 dauerhaft angetrieben sein oder abwechselnd eingeschaltet oder ausgeschaltet zu werden. Die Ausgestaltung, bei der die Pumpe 13 dauerhaft eingeschaltet ist, lässt sich mit der beweglichen Lochblende kombinieren. Die Ausgestaltung, bei der die Pumpe 13 eingeschaltet und ausgeschaltet wird, vermeidet die Notwendigkeit, eine Lochblende oder einen sonstigen Verschluss vorzusehen.

Bei der zweiten Ausführungsform (Figur 2) trennt eine gasdurchlässige Membrane 8 die Messkammer 9 von der Umgebung, sodass die Gasprobe G aus der Umgebung durch die Membrane 8 hindurch in die Messkammer 9 hinein und auch wieder durch die Membrane 8 hindurch aus der Messkammer 9 heraus fließen kann. Bei der gezeigten zweiten Ausführungsform tritt die Membrane 8 an die Stelle des Einlasses E und des Auslasses A der ersten Ausführungsform (Figur 1). Auch bei der zweiten Ausführungsform kann eine Pumpe 13 (in Figur 2 nicht gezeigt) die Gasprobe G ansaugen, diesmal durch die Membrane 8 hindurch. Bevorzugt verhindert ein nicht gezeigter Flammschutz, beispielsweise ein metallisches Gitter, dass Flammen aus der Messkammer 9 durch die Membrane 8 hindurch in die Umgebung schlagen können.

Auch bei der zweiten Ausführungsform kann eine Lochblende (nicht gezeigt) relativ zum Gehäuse 5 beweglich sein und je nach Stellung die Messkammer 9 fluiddicht von der Umgebung trennen oder freigeben.

Falls nicht anders erwähnt, bezieht sich die nachfolgende Beschreibung auf beide Ausführungsformen.

Im Inneren der Messkammer 9 ist ein Halbleiter-Sensor 1 angeordnet. Der Halbleiter-Sensor 1 umfasst ein Halbleiter-Bauteil 10 sowie ein Heizelement 11. Das Halbleiter-Bauteil 10 fungiert als das Sensor-Bauteil im Sinne der Ansprüche. Das Halbleiter-Bauteil 10 ist elektrisch leitend und ist bevorzugt aus einem Metalloxid, besonders bevorzugt aus einem Halbleiter, aufgebaut, beispielsweise aus Zinndioxid ($SnO_2$). In Figur 1 und Figur 2 ist das Halbleiter-Bauteil 10 als ein Draht dargestellt, dies ist aber als ein Symbol in einem Ersatzschaltbild zu verstehen und nur eine von mehreren möglichen Realisierungsformen. Elektrische Kontaktierungen für das Halbleiter-Bauteil 10 und für nachfolgend beschriebene weitere Bestandteile der Gasdetektionsvorrichtung 100 sind angedeutet.

Eine Gasprobe G in der Messkammer 9 erreicht das Halbleiter-Bauteil 10. Die Gasprobe G wirkt auf das Halbleiter-Bauteil 10 ein und beeinflusst dessen elektrischen Widerstand R. Im Ausführungsbeispiel ist also der elektrische Widerstand R die beeinflusste und messbare Detektionsgröße. Die chemische Einwirkung ist im Ausführungsbeispiel dergestalt, dass der elektrische Widerstand R des Halbleiter-Bauteils 10 umso geringer ist, je höher die Konzentration des brennbaren Zielgases in der Gasprobe G und damit in der Messkammer 9 ist. Ein Grund: ein brennbares Zielgas hat häufig einen höheren Wärmeleitwert als Atemluft, und daher kühlt brennbares Zielgas in der Messkammer 9 das Halbleiter-Bauteil 10 ab.

Eine bevorzugte Funktionsweise des Halbleiter-Bauteils 10 wird nachfolgend am Beispiel von Zinndioxid ($SnO_2$) als dem Material des Halbleiter-Bauteils 10 beschrieben: Die elektrische Leitfähigkeit und damit der elektrische Widerstand R hängen von der Anzahl der freien Elektronen (Ladungsträger) im Halbleiter-Bauteil 10 ab. Die Oberfläche von $SnO_2$-Kristallen weist $O_2$-Fehlstellen auf, sodass die Elektronen, welche benachbarte Sn-Atome zur Verfügung stellen, keinen Partner finden. Diese Elektronen sind frei beweglich. Die Anzahl der frei beweglichen Elektronen beeinflusst die elektrische Leitfähigkeit und damit den elektrischen Widerstand R des Halbleiter-Bauteils 10. Das Halbleiter-Bauteil 10 adsorbiert an seiner Oberfläche Sauerstoff aus der Umgebung. Dadurch wird wenigstens ein Teil der $O_2$-Fehlstellen durch Adsorption von umgebendem Sauerstoff besetzt, und die zuvor freien Elektronen werden gebunden. Das Halbleiter-Bauteil 10 wird erhitzt betrieben, beispielsweise mithilfe des Heizelements 11. Ein brennbares Zielgas wird an der Oberfläche des Halbleiter-Bauteils 10 oxidiert, wobei Sauerstoff, der so wie gerade beschrieben adsorbiert wurde, wieder desorbiert wird. Daher nimmt die Dichte von Ladungsträgern in Form von freien Elektronen wieder zu. Je höher der Anteil von Sauerstoff in der Umgebung des Halbleiter-Bauteils 10 ist, desto mehr freie Elektronen verbinden sich mit Sauerstoff, und desto größer ist der elektrische Widerstand R. Die Oxidation von brennbarem Zielgas verringert also die Menge des Sauerstoffs, welchen das Halbleiter-Bauteil 10 adsorbieren kann. Bei ansonsten gleichbleibenden Umgebungsbedingungen ist der elektrische Widerstand R des Halbleiter-Bauteils 10 daher umso kleiner, je größer die Konzentration eines brennbaren Zielgases in der Messkammer 9 ist. Diese Eigenschaft wird erfindungsgemäß ausgenutzt.

An jedem Abtast-Zeitpunkt einer vorgegebenen Abfolge von Abtast-Zeitpunkten wird erneut ein Maß für den aktuellen elektrischen Widerstand R des Halbleiter-Bauteils 10 gemessen. Beispielsweise misst ein Spannungs-Sensor 25 die elektrische Spannung U, die am Halbleiter-Bauteil 10 anliegt. Ein Stromstärken-Sensor 24 misst die Stärke I des elektrischen Stroms, der durch das Halbleiter-Bauteil 10 fließt. Aus der Spannung U und der Stromstärke I wird der elektrische Widerstand R = U/I hergeleitet. Wie gerade dargelegt, korreliert dieser elektrische Widerstand R mit der Konzentration des Zielgases in der Messkammer 9 und hängt außerdem von Umgebungsbedingungen ab, insbesondere von der Temperatur in der Messkammer 9.

Das Heizelement 11 hat Ausführungsbeispiel die Form eines elektrischen Widerstands und steht in einem thermischen Kontakt mit dem Halbleiter-Bauteil 10, sodass die Temperatur des Heizelements 11 ausreichend genau mit der Temperatur des Halbleiter-Bauteils 10 übereinstimmt. Das Aufheizen des Halbleiter-Bauteils 10 bewirkt so wie oben beschrieben, dass brennbares Zielgas oxidiert und Sauerstoff desorbiert wird.

Variable Umgebungsbedingungen, insbesondere Temperatur, Feuchte und Luftdruck, beeinflussen ebenfalls die elektrische Leitfähigkeit des Halbleiter-Bauteils 10. Eine mögliche Ursache ist, dass Umgebungsbedingungen die Oberflächentemperatur des Halbleiter-Bauteils 10 verändern können, beispielsweise die Temperatur der äußeren Oberfläche des Halbleiter-Sensors 1.

Nachfolgend wird beschrieben, wie der Einfluss dieser Umgebungsbedingungen auf den elektrischen Widerstand R bis zu einem gewissen Grad rechnerisch kompensiert wird. Weil dieser Einfluss kompensiert wird, lassen sich gemessene Werte des elektrischen Widerstands R - allgemein: der Detektionsgröße - verwenden, um die gesuchte Zielgas-Konzentration zu ermitteln.

In einer bevorzugten Realisierung der Erfindung wird die Temperatur des Halbleiter-Bauteils 10 konstant gehalten, und zwar auf einer Temperatur oberhalb jeder beim Einsatz möglichen Umgebungstemperatur. Dadurch wird der Einfluss der Umgebungstemperatur auf den elektrischen Widerstand R des Halbleiter-Bauteils 10 verringert.

Bevorzugt regelt das Steuergerät 12 die Temperatur des Heizelements 11 mit dem Regelungsziel, dass die Temperatur des Heizelements 11 trotz variabler Umgebungsbedingungen konstant bleibt und daher der Eintrag von Wärmeenergie pro Zeiteinheit, den das Heizelement 11 auf das Halbleiter-Bauteil 10 ausübt, ebenfalls konstant bleibt, und zwar auch bei variierender Umgebungstemperatur. Um bei Bedarf die Wärmeenergie, die das Heizelement 11 abgibt, zu verändern, verändert das Steuergerät 12 in einer Ausgestaltung die elektrische Spannung U, die am Heizelement 11 anliegt. In einer anderen Ausgestaltung verändert das Steuergerät 12 die Stärke I des Stroms, der durch das Heizelement 11 fließt. Diese beiden Ausgestaltungen lassen sich miteinander kombinieren. Weil die Temperatur des Halbleiter-Bauteils 10 oberhalb der Umgebungstemperatur liegen soll, reicht diese einseitige Temperatur-Regelung mit dem Heizelement 11 als dem Stellglied aus. Zwar möglich, aber in der Regel nicht erforderlich ist, das Heizelement 11 in gesteuerter Weise zu kühlen.

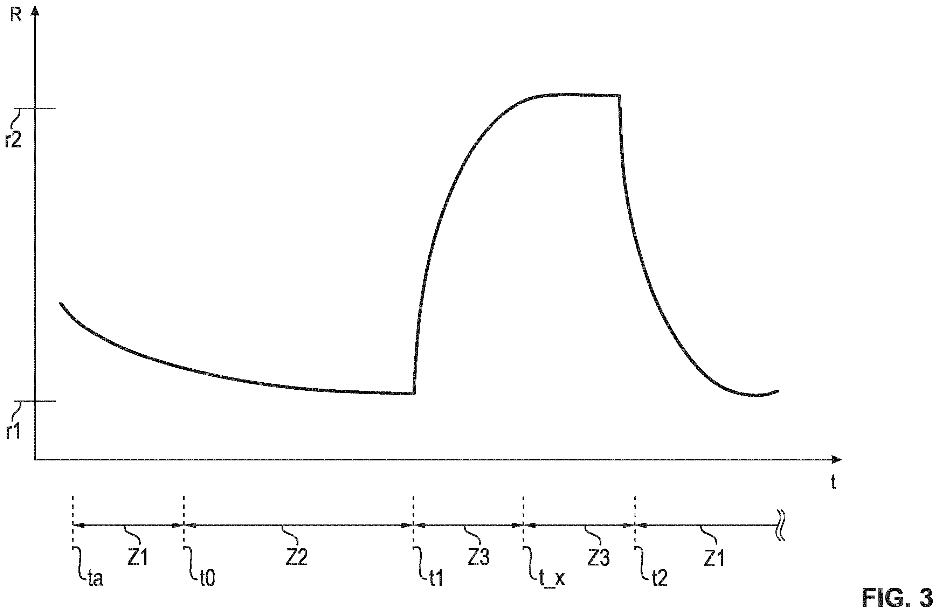

Der elektrische Widerstand des Halbleiter-Bauteils 10 hängt auch bei annähernd gleichbleibender Temperatur von Umgebungsbedingungen ab, insbesondere vom Sauerstoffgehalt in der Umgebung, manchmal auch von der Feuchte. Daher wird der elektrische Widerstand R des Halbleiter-Bauteils 10 mindestens an einem ersten Abtast-Zeitpunkt t1 und an einem nachfolgenden zweiten Abtast-Zeitpunkt t2 gemessen. Am ersten Abtast-Zeitpunkt t1 beginnt ein Mess-Zeitraum Z3, und am zweiten Abtast-Zeitpunkt t2 endet dieser Mess-Zeitraum Z3, vgl. Figur 3. Möglich ist, den elektrischen Widerstand R zusätzlich mindestens einmal an einem Abtast-Zeitpunkt t_x zwischen diesen beiden Abtast-Zeitpunkten t1 und t2 zu messen. Im Ausführungsbeispiel stimmt dieser Mess-Zeitraum Z3 mit einem nachfolgend beschriebenen Oxidierungs-Zeitraum überein. Sowohl der Mess-Zeitraum als auch der Oxidierungs-Zeitraum beginnen am ersten Zeitpunkt t1 und enden am zweiten Zeitpunkt t2. Daher wird auch für den Oxidierungs-Zeitraum das Bezugszeichen Z3 verwendet. Im Ausführungsbeispiel fungiert der erste Zeitpunkt t1 als der Detektions-Zeitpunkt, der zweite Zeitpunkt t2 als der Referenz-Zeitpunkt.

In der Darstellung von Figur 3 sind auf der x-Achse die Zeit t und auf der y-Achse der gemessene Widerstand R des Halbleiter-Bauteils 10 aufgetragen. Am ersten Abtast-Zeitpunkt (Detektions-Zeitpunkt) t1 stimmt die Konzentration des brennbaren Zielgases in der Gasprobe G und somit in der Messkammer 9 ausreichend genau mit der Zielgas-Konzentration in der Umgebung der Gasdetektionsvorrichtung 100, also in dem zu überwachenden Bereich, überein. Am zweiten Abtast-Zeitpunkt (Referenz-Zeitpunkt) t2 befindet sich praktisch kein brennbares Zielgas in der Messkammer 9, weil vorhandenes Zielgas im Zeitraum Z3 oxidiert wurde. Die Messung am zweiten Abtast-Zeitpunkt t2 fungiert also als eine Referenz-Messung oder eine Nullpunkt-Messung.

Um am zweiten Abtast-Zeitpunkt t2 eine solche Referenz-Messung durchführen zu können, wird während des Oxidierungs-Zeitraums Z3 das brennbare Zielgas in der Messkammer 9 beseitigt, sodass am zweiten Zeitpunkt t2 kein brennbares Zielgas in der Messkammer 9 vorhanden ist. Dieses Beseitigen wird durchgeführt, indem ein Oxidierungs-Bauteil 2 das brennbare Zielgas, welches sich als Bestandteil der Gasprobe G in der Messkammer 9 befindet, oxidiert. Selbstverständlich ist es möglich, dass im zu überwachenden Bereich kein brennbares Zielgas vorhanden ist und daher die Messkammer 9 bereits am ersten Zeitpunkt t1 frei von brennbarem Zielgas ist.

Bevorzugt wird eine Konzentrations-Schranke als obere Schranke für die zu erwartende Konzentration des Zielgases in dem zu überwachenden Bereich vorgegeben. Diese Konzentrations-Schranke sowie das konstruktionsbedingte Volumen der Messkammer 9 legen die maximal mögliche Menge von brennbarem Zielgas in der Messkammer 9 fest. Diese maximal mögliche Zielgas-Menge ist so gering, dass genügend Sauerstoff in der Messkammer 9 vorhanden ist, um das gesamte Zielgas in der Messkammer 9 zu oxidieren.

Der Oxidierungs-Zeitraum Z3 ist einerseits so lang, dass im Verlaufe des Oxidierungs-Zeitraums Z3 das gesamte brennbare Zielgas in der Messkammer 9 oxidiert wird - vorausgesetzt die Konzentration des Zielgases liegt unterhalb der Konzentrations-Schranke. Andererseits ist der Oxidierungs-Zeitraum Z3 bevorzugt so kurz wie möglich, um mit einer möglichst hohen Frequenz die Schritte wiederholen zu können, brennbares Zielgas zu oxidieren und den elektrischen Widerstand R des Halbleiter-Bauteils 10 zweimal zu messen.

In einer Abweichung wird nicht notwendigerweise eine Konzentrations-Schranke vorgegeben. Der Mess-Zeitraum und Oxidierungs-Zeitraum Z3 wird beendet, wenn Messungen das Ergebnis haben, dass der elektrische Widerstand R gleich bleibt - genauer gesagt: wenn die Steigung des zeitlichen Verlaufs des elektrischen Widerstands R unter einer vorgegebenen Schranke bleibt. Dies zeigt an, dass alles brennbare Zielgas oder wenigstens ein vorgegebener Anteil des Zielgases in der Messkammer 9 oxidiert ist.

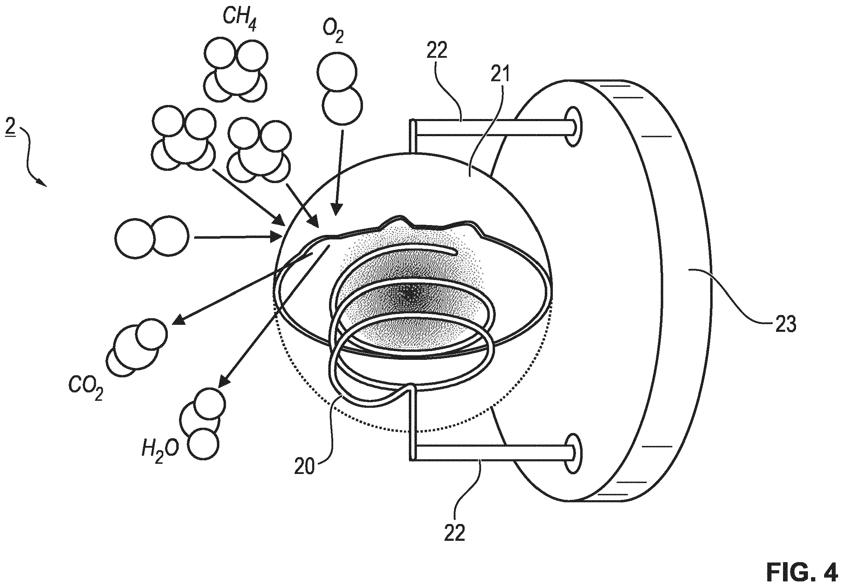

Das brennbare Zielgas in der Messkammer 9 wird von dem Oxidierungs-Bauteil 2 oxidiert. Figur 4 zeigt eine bevorzugte Ausgestaltung des Oxidierungs-Bauteils 2. In diesem Beispiel ist das brennbare Zielgas Methan (CH4). Das Oxidierungs-Bauteil 2 bewirkt die chemische Reaktion

$$CH4 + 2*O2 \rightarrow 2*H2O + CO2.$$

Diese chemische Reaktion ist in Figur 4 angedeutet. Zwar wird beim Oxidieren des Zielgases Sauerstoff gebunden. Die optionale Konzentrations-Schranke ist aber so vorgegeben, dass die Verringerung des Gehalts an $O_2$-Molekülen in der Messkammer 9 aufgrund des Oxidierens so gering ist, dass der elektrische Widerstand R des Halbleiter-Bauteils 10 dadurch nur in vernachlässigbarer Weise verändert wird.

In der in Figur 4 gezeigten bevorzugten Ausgestaltung ist das Oxidierungs-Bauteil 2 als ein Pellistor ausgestaltet und umfasst

- ein spiralförmiges heizendes Segment 20,
- eine bevorzugt kugelförmige Ummantelung 21 um das heizende Segment 20,
- zwei elektrische Kontaktierungen 22 und
- eine Platte 23.

An das heizende Segment 20 wird eine elektrische Spannung angelegt. Dadurch wird das heizende Segment 20 auf eine Betriebstemperatur erhitzt, die zwischen 300 °C und 700 °C liegt, bevorzugt zwischen 400 °C und 550 °C. Das heizende Segment 20 steht in einem thermischen Kontakt mit der Ummantelung 21, sodass auch die Ummantelung 21 erhitzt wird.

Diese Temperatur alleine würde aber noch nicht ausreichen, um in ausreichendem Maße brennbares Zielgas zu oxidieren. Eine höhere Temperatur verbraucht mehr elektrische Energie und erhöht das Risiko, dass ein Zielgas in der Messkammer 9 schlagartig verbrennt oder sogar explodiert. Um trotz einer Temperatur von bevorzugt unter 550 °C alles brennbare Zielgas in der Messkammer 9 zu oxidieren, ist in die Ummantelung 21 ein katalytisches Material eingelassen, beispielsweise Platin oder ein Platinoxid. Bevorzugt ist die Ummantelung 21 porös, sodass die thermisch wirksame Oberfläche der Ummantelung 21 größer ist als bei einer glatten Oberfläche.

Zwischen dem Halbleiter-Sensor 1 und dem Oxidierungs-Bauteil 2 ist eine thermische Barriere 4 angeordnet, die in Figur 1 und Figur 2 schematisch gezeigt ist. Diese thermische Barriere 4 reduziert den thermischen Einfluss des Oxidierungs-Bauteils 2 auf den Halbleiter-Sensor 1 und reduziert daher die Gefahr, dass der elektrische Widerstand R des Halbleiter-Bauteils 10 in nennenswerter Weise durch das erhitzte Oxidierungs-Bauteil 2 und / oder durch das Einschalten und durch das Ausschalten des Oxidierungs-Bauteils 2 verändert wird, was zu einer falschen Messung könnte. Zwischen dem Gehäuse 5 und der thermischen Barriere 4 tritt aber mindestens eine Öffnung auf, bevorzugt eine umlaufende Öffnung, sodass die Gasprobe G durch die gesamte Messkammer 9 fließen kann. Dies ist gewünscht, damit das Oxidierungs-Bauteil 2 das gesamte Zielgas in der Messkammer 9 oxidieren kann, auch das Zielgas hinter der thermischen Barriere 4, und um den gemessenen elektrischen Widerstand R des Halbleiter-Bauteils 10 als Maß für die gesuchte Zielgas-Konzentration verwenden zu können.

Das Steuergerät 12 vermag das Oxidierungs-Bauteil 2 einzuschalten und auszuschalten. In einer ersten Ausgestaltung der Erfindung bewirkt das Steuergerät 12, dass das Oxidierungs-Bauteil 2 in jedem Oxidierungs-Zeitraum Z3 eingeschaltet und außerhalb eines Oxidierungs-Zeitraums Z3 ausgeschaltet ist. Weil das Oxidierungs-Bauteil 2 eine relativ geringe thermische Masse aufweist, erreicht es nach dem Einschalten schnell die Betriebstemperatur zwischen 300 °C und 700 °C und kühlt nach einem Ausschalten rasch auf die Temperatur in der Messkammer 9 ab. Diese gewollte starke und in der Regel oszillierende Temperaturänderung führt in der Regel zwangsläufig zu einem oszillierenden Eintrag von Wärmeenergie pro Zeiteinheit durch das Oxidierungs-Bauteil 2 auf die äußere Oberfläche des Halbleiter-Sensors 1. Die oszillierende Temperatur verändert in der Regel den elektrischen Widerstand R des Halbleiter-Bauteils 10 und könnte daher zu fehlerhaften Messungen führen.

Das Oxidierungs-Bauteil 2, dessen Temperatur bei der ersten Ausgestaltung oszilliert, kann einen unerwünschten thermischen Einfluss auf das Halbleiter-Bauteil 10 nehmen. Um diesen thermischen Einfluss in der Messkammer 9 zu verringern, ist in der Messkammer 9 zusätzlich ein ansteuerbares Heizelement 3 angeordnet, und zwar auf derselben Seite der therm ischen Barriere 4 wie das Oxidierungs-Bauteil 2.

Das Steuergerät 12 vermag gemäß der ersten Ausgestaltung nicht nur das Oxidierungs-Bauteil 2, sondern auch das Heizelement 3 einzuschalten und auszuschalten. Das eingeschaltete Heizelement 3 bewirkt idealerweise den gleichen Eintrag von Wärmeenergie pro Zeiteinheit in das Halbleiter-Bauteil 10 wie das eingeschaltete Oxidierungs-Bauteil 2. Die Wirkung: Das Oxidierungs-Bauteil 2 nimmt einen ähnlichen thermischen Einfluss auf das Halbleiter-Bauteil 10 wie das Heizelement 3. Die Gefahr, dass die aktuelle Temperatur des Oxidierungs-Bauteils 2 die Messergebnisse des Halbleiter-Sensors 1 verfälscht, wird reduziert.

In einer Ausgestaltung umfasst das Heizelement 3 genauso wie das Oxidierungs-Bauteil 2 ein spiralförmiges heizendes Segment 20, eine Ummantelung 21 und elektrische Kontaktierungen 22, jedoch kein katalytisches Material in der Ummantelung 21. Daher vermag das eingeschaltete Heizelement 3 auch mit erhitztem heizenden Segment 20 kein brennbares Zielgas in der Messkammer 9 zu oxidieren.

Zeitlich vor oder auch nach dem Oxidierungs-Zeitraum (= Mess-Zeitraum) Z3 ist ein Einlass-Zeitraum Z1 vorhanden. Wenigstens in diesem Einlass-Zeitraum Z1 kann eine Gasprobe G aus dem Bereich in die Messkammer 9 fließen, insbesondere indem die Gasprobe G von der Pumpe 13 angesaugt wird und / oder in die Messkammer 9 diffundiert. Zwischen dem Einlass-Zeitraum Z1 und dem Oxidierungs-Zeitraum Z3 ist bei der ersten Ausgestaltung ein Heiz-Zeitraum Z2 angeordnet, vgl. Figur 3.

In jedem Oxidierungs-Zeitraum Z3 ist das Oxidierungs-Bauteil 2 eingeschaltet, und das Heizelement 3 ist ausgeschaltet. Im Heiz-Zeitraum Z2 ist das Heizelement 3 eingeschaltet, und das Oxidierungs-Bauteil 2 ist ausgeschaltet. Das Heizelement 3 heizt die Gasprobe G in der Messkammer 9 auf, sodass beim Übergang von einem Heiz-Zeitraum Z2 zu einem Oxidierungs-Zeitraum (= Mess-Zeitraum Z3) in der Messkammer 9 keine abrupte Temperaturänderung auftritt. Dank des Heizelements 3 ist der Eintrag von thermischer Energie pro Zeiteinheit in das Halbleiter-Bauteil 10 im Heiz-Zeitraum Z2 etwa gleich dem Eintrag im Mess-Zeitraum Z3. Insbesondere variiert der Eintrag von Heizenergie in den Halbleiter-Sensor 1 über der Zeit weniger verglichen mit einem Zustand ohne ein Heizelement 3. Im Einlass-Zeitraum Z1 sind bevorzugt sowohl das Oxidierungs-Bauteil 2 als auch das Heizelement 3 ausgeschaltet.

In einer Ausgestaltung wird vorab eine Justierung durchgeführt, um eine Soll-Betriebstemperatur Temp_Soll(3) des Heizelements 3 festzulegen. Das Heizelement 3 erreicht nach dem Einschalten diese Soll-Betriebstemperatur Temp_Soll(3). Das Ziel bei der Justierung ist, dass der Eintrag von Wärmeenergie pro Zeiteinheit auf den Halbleiter-Sensor 1, den das eingeschaltete Heizelement 3 bewirkt, gleich dem Eintrag von Wärmeenergie pro Zeiteinheit ist, den das eingeschaltete Oxidierungs-Bauteil 2 bewirkt. Außer der Soll-Betriebstemperatur Temp_Soll(3) lassen sich auch der Abstand dist(3) zwischen dem Heizelement 3 und dem Halbleiter-Sensor 1 sowie der Abstand dist(2) zwischen dem Oxidierungs-Bauteil 2 und dem Halbleiter-Sensor 1 verändern. Für die Justierung wird ein Zustand hergestellt, bei dem die Messkammer 9 frei von brennbarem Zielgas ist. Die Soll-Betriebstemperatur Temp_Soll(3) und der Abstand werden so eingestellt, dass die Detektionsgröße, hier also der elektrische Widerstand R, des Halbleiter-Bauteils 10 bei eingeschaltetem Oxidierungs-Bauteil 2 und ausgeschaltetem Heizelement 3 genauso groß ist wie bei ausgeschaltetem Oxidierungs-Bauteil 2 und eingeschaltetem Heizelement 3.

Figur 3 veranschaulicht einen beispielhaften zeitlichen Verlauf beim Betrieb der Gasdetektionsvorrichtung 100. Auf der x-Achse ist die Zeit t aufgetragen, auf der y-Achse der elektrische Widerstand R des Halbleiter-Bauteils 10. Die nachfolgende Beschreibung bezieht sich auf die erste Ausführungsform gemäß Figur 1.

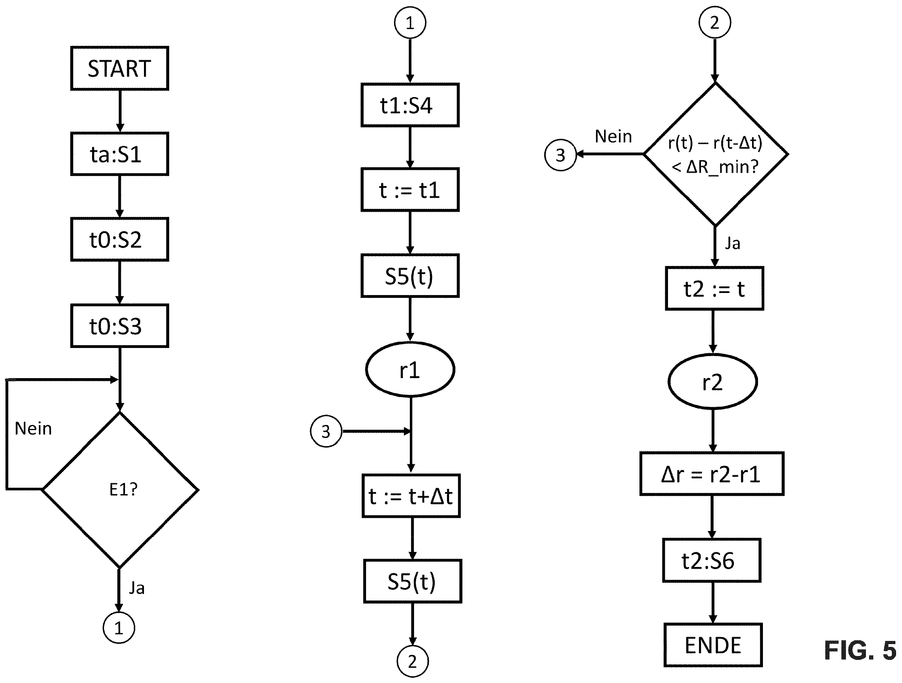

Mindestens einmal wird eine Abfolge durchgeführt, die aus dem Einlass-Zeitraum Z1, dem nachfolgenden Heiz-Zeitraum Z2 und dem nachfolgenden Mess-Zeitraum (= Oxidierungs-Zeitraum Z3) besteht. Bevorzugt wird diese Abfolge mit den drei Zeiträumen Z1, Z2, Z3 wiederholt durchgeführt, während die Gasdetektionsvorrichtung 100 verwendet wird. Figur 5 zeigt ein beispielhaftes Flussdiagramm für eine derartige Abfolge beim Betrieb der Gasdetektionsvorrichtung 100.

Der Einlass-Zeitraum Z1 beginnt zum Zeitpunkt ta. Im Einlass-Zeitraum Z1 sind die Ventile 6 und 7 geöffnet, und die optionale Pumpe 13 wird eingeschaltet. Die Messkammer 9 wird ausgespült und mit einer neuen Gasprobe G gefüllt. Dies bedeutet: Die zuvor in der Messkammer 9 vorhandene Gasprobe G fließt durch den Auslass A aus der Messkammer 9 heraus, und die nunmehr zu untersuchende Gasprobe G fließt aus dem zu überwachenden Bereich durch den Einlass E hindurch in die Messkammer 9. In einer Ausgestaltung ist die optionale Pumpe 13 eingeschaltet und fördert die Gasprobe G aus der Umgebung in die Messkammer 9 hinein. Möglich ist auch, dass die zu untersuchende Gasprobe G aus dem Bereich in die Messkammer 9 hinein diffundiert.

In Figur 5 bedeutet ta:S1 den Schritt, dass zum Zeitpunkt ta die Ventile 6 und 7 geöffnet werden, die Pumpe 13 eingeschaltet wird und die Messkammer 9 ausgespült wird. Das Heizelement 3 und das Oxidierungs-Bauteil 2 sind ausgeschaltet.

Der Einlass-Zeitraum Z1 ist so lang, dass nach dem Ende des Einlass-Zeitraums Z1 die Konzentration von brennbarem Zielgas in der Messkammer 9 etwa gleich der Zielgas-Konzentration in der Umgebung und daher in dem zu überwachenden Bereich ist. Insbesondere ist der Einlass-Zeitraum Z1 so lang, dass dann, wenn in der Messkammer 9 kein brennbares Zielgas detektiert ist, fest steht, dass auch in der Umgebung kein brennbares Zielgas oberhalb einer Nachweisgrenze vorhanden ist. In einer Ausgestaltung ist die Dauer des Einlass-Zeitraums Z1 fest vorgegeben.

Im Einlass-Zeitraum Z1 ist das Oxidierungs-Bauteil 2 ausgeschaltet. In einer Realisierungsform ist im Einlass-Zeitraum Z1 auch das Heizelement 3 ausgeschaltet, wodurch elektrische Energie eingespart wird. In einer anderen Realisierungsform ist oder wird das Heizelement im Einlass-Zeitraum Z1 eingeschaltet, was häufig einen kürzeren Heiz-Zeitraum Z2 ermöglicht und dadurch Zeit einspart.

Zum Zeitpunkt t0 ist der Einlass-Zeitraum Z1 beendet, und der nachfolgende Heiz-Zeitraum Z2 beginnt. Im Heiz-Zeitraum Z2 bleibt das Oxidierungs-Bauteil 2 ausgeschaltet. Das Steuergerät 12 löst zum Zeitpunkt t0 folgenden Ereignisse aus:

- Die Ventile 6 und 7 werden geschlossen, sodass die Messkammer 9 von der Umgebung getrennt ist.
- Die optionale Pumpe 13 wird ausgeschaltet.
- Das Heizelement 3 wird eingeschaltet.

In Figur 5 bedeutet t0:S2 den Schritt, dass zum Zeitpunkt t0 die Ventile 6 und 7 geschlossen werden und die Pumpe 13 ausgeschaltet wird. t0:S3 bedeutet den Schritt, dass zum Zeitpunkt t0 das Heizelement 3 eingeschaltet wird. Das Oxidierungs-Bauteil 2 bleibt ausgeschaltet.

Der Heiz-Zeitraum Z2, in dem das Heizelement 3 eingeschaltet ist, ist so lang, dass das Heizelement 3 im Heiz-Zeitraum Z2 auf die Soll-Betriebstemperatur Temp_Soll(3) aufgeheizt wird. Die Soll-Betriebstemperatur Temp_Soll(3) wurde wie oben beschrieben in einer vorhergehenden Justierung ermittelt und bewirkt den gleichen Eintrag von Wärmeenergie pro Zeiteinheit wie später das eingeschaltete Oxidierungs-Bauteil 2.

In einer Ausgestaltung ist die Soll-Betriebstemperatur Temp_Soll(3) des Heizelements 3 vorgegeben. Die aktuelle Temperatur Temp(3) des Heizelements 3 wird gemessen. Beispielsweise wird der aktuelle elektrische Widerstand des Heizelements 3 gemessen. Bekanntlich korreliert der elektrische Widerstand eines Metalls mit dessen Temperatur, so dass der Widerstand ein Maß für die Temperatur ist.

In Figur 5 bezeichnet E1? die Entscheidung, ob der Heiz-Zeitraum Z2 bereits verstrichen ist oder nicht, ob also das Heizelement 3 die Soll-Betriebstemperatur Temp_Soll(3) erreicht hat.

Zum Zeitpunkt t1 (Detektions-Zeitpunkt) ist der Heiz-Zeitraum Z2 beendet, und der Mess-Zeitraum Z3 beginnt. Das Steuergerät 12 löst zum Zeitpunkt t1 folgende Ereignisse aus:

- Der elektrische Widerstand R des Halbleiter-Bauteils 10 wird gemessen. Der zu t1 gemessene Wert des elektrischen Widerstands wird mit r1 bezeichnet.
- Das Heizelement 3 wird ausgeschaltet.
- Das Oxidierungs-Bauteil 2 wird eingeschaltet.

Im Mess-Zeitraum Z3 bleibt das Heizelement 3 ausgeschaltet. Die Ventile 6 und 7 bleiben geschlossen, und die Pumpe 13 bleibt ausgeschaltet. Das im Oxidierungs-Zeitraum (= Mess-Zeitraum) Z3 eingeschaltete Oxidierungs-Bauteil 2 oxidiert das oder jedes brennbare Zielgas in der Messkammer 9.

Selbstverständlich ist es möglich, dass sich in dem zu überwachenden Bereich und damit auch in der Messkammer 9 kein brennbares Zielgas befindet und daher das erhitzte Oxidierungs-Bauteil 2 kein Oxidieren durchführt.

Im Ausführungsbeispiel ist der erste Zeitpunkt t1 sowohl das Ende des Heiz-Zeitraums Z2 als auch der Beginn des Mess-Zeitraums (= Oxidierungs-Zeitraums) Z3. In Figur 5 bedeutet t1:S4 den Schritt, dass zum Zeitpunkt t1 das Heizelement 3 ausgeschaltet und das Oxidierungs-Bauteil 2 eingeschaltet werden. S5(t) bedeutet, dass zum Zeitpunkt t der elektrische Widerstand R des Halbleiter-Bauteils 10 gemessen wird. Anfänglich wird der Zeitpunkt t auf t1 gesetzt. Die Messung S5(t1) zum ersten Zeitpunkt t1 liefert den Widerstands-Wert r1.

Zum Zeitpunkt t2 (Referenz-Zeitpunkt) sind der Mess-Zeitraum Z3 und damit die Abfolge bestehend aus den Zeiträumen Z1, Z2, Z3 beendet.

Das Steuergerät 12 löst zum Zeitpunkt t2 folgenden Ereignisse aus:

- Der elektrische Widerstand R des Halbleiter-Bauteils 10 wird erneut gemessen. Der Messung S5(t2) zum zweiten Zeitpunkt t2 liefert einen Wert des elektrischen Widerstands R, der mit r2 bezeichnet wird.
- Das Oxidierungs-Bauteil 2 wird ausgeschaltet.

In dem beispielhaften Flussdiagramm ist der Oxidierungs-Zeitraum Z3 zwischen t1 und t2 nicht fest vorgegeben. Vielmehr wird der elektrische Widerstand R an den Zeitpunkten t1, t1+$\Delta$t, t1+2*$\Delta$t, ... gemessen, wobei $\Delta$t ein fest

vorgegebener Abstand ist. Weil das Oxidierungs-Bauteil 2 brennbares Zielgas in der Messkammer 9 verbrennt, wird der elektrische Widerstand R immer größer. Mit r(t) wird der Widerstands-Wert zum Zeitpunkt t bezeichnet. Berechnet wird die Differenz zwischen den Widerstands-Werten an zwei unmittelbar aufeinanderfolgenden Zeitpunkten t-$\Delta$t und t, also die Differenz r(t) - r(t-$\Delta$t).

Im gezeigten Beispiel kann während des Mess-Zeitraums Z3 kein brennbares Zielgas von außen in die Messkammer 9 gelangen. Falls die Differenz r(t) - r(t-$\Delta$t) kleiner als eine vorgegebene Schranke $\Delta$R_min ist, so ist praktisch alles brennbare Zielgas in der Messkammer 9 oxidiert. Der Zeitpunkt t, an dem dies festgestellt wird, wird als der Zeitpunkt t2 verwendet, an dem der Mess-Zeitraum Z3 beendet ist. Der zuletzt gemessene Widerstands-Wert r(t) wird als der Wert r2 = r(t2) verwendet.

Im gezeigten Beispiel wird die Differenz r(t) - r(t-$\Delta$t) zwischen den beiden jüngsten Widerstands-Werten verwendet. Allgemein wird die Steigung des elektrischen Widerstands R als Funktion der Zeit berechnet, wofür die Zeitreihe r(t1), r(t1+$\Delta$t), r(t1+2*$\Delta$t), ... verwendet wird. Wenn diese Steigung unter einer vorgegebenen Schranke liegt, wird der Zeitpunkt der jüngsten Messung als zweiter Zeitpunkt t2 verwendet.

In Figur 5 bedeutet t2:S6, dass zum Zeitpunkt t2 das Oxidierungs-Bauteil 2 ausgeschaltet wird.

Eine nachfolgende Abfolge beginnt. In Figur 3 ist der Einlass-Zeitraum Z1 der nachfolgenden Abfolge angedeutet. Optional werden zum zweiten Zeitpunkt t2 die Ventile 6 und 7 wieder geöffnet, und die Pumpe 13 wird wieder eingeschaltet.

In der gerade beschriebenen ersten Ausgestaltung ist das Oxidierungs-Bauteil 2 nur während des Oxidierungs-Zeitraum Z3 eingeschaltet und ansonsten ausgeschaltet. Nachfolgend wird eine alternative zweite Ausgestaltung beschrieben. Bei dieser zweiten Ausgestaltung ist das Oxidierungs-Bauteil 2 nicht nur im Oxidierungs-Zeitraum Z3 eingeschaltet, sondern wenigstens auch im Einlass-Zeitraum Z1. Optional bleibt das Oxidierungs-Bauteil 2 während des gesamten Betriebs der Gasdetektionsvorrichtung 100 eingeschaltet und wird nur in einem Ruhezustand der Gasdetektionsvorrichtung 100 ausgeschaltet. In vielen Fällen spart der Ausgestaltung, das Oxidierungs-Bauteil 2 eingeschaltet zu lassen, ein Heizelement 3 ein. An den Einlass-Zeitraum Z1 kann sich direkt der Oxidierungs-Zeitraum Z3 anschließen, ein Heiz-Zeitraum Z2 wird also nicht benötigt. Die zweite Ausgestaltung lässt sich aber auch in Kombination mit einem Heizelement 3 verwenden, welches mögliche Schwankungen in dem Eintrag von Wärmeenergie, den das Oxidierungs-Bauteil 2 auf das Halbleiter-Bauteil 10 bewirkt, bis zu einem gewissen Grade kompensiert.

Bei der zweiten Ausgestaltung ist die Messkammer 9 im Einlass-Zeitraum Z1 geöffnet, sodass eine Gasprobe G aus dem zu überwachenden Bereich in die Messkammer 9 fließen kann. Im Oxidierungs-Zeitraum Z3 ist die Messkammer 9 geschlossen und damit fluiddicht gegen den zu überwachenden Bereich abgeschlossen, sodass im Oxidierungs-Zeitraum Z3 kein Zielgas in die Messkammer 9 fließen kann, auch wenn im Bereich brennbares Zielgas vorhanden ist.

Den Vorgang, die Messkammer 9 zu öffnen und zu schließen und dadurch wahlweise in einem geöffneten oder in einem geschlossenen Zustand zu betreiben, lässt sich beispielsweise auf eine der folgenden Weisen realisieren:

- Die Pumpe 13 wird eingeschaltet und ausgeschaltet. Insbesondere ist die Pumpe 13 im Einlass-Zeitraum Z1 eingeschaltet und im oxidieren Zeitraum Z3 ausgeschaltet.
- Das Ventil 6 am Einlass E wird geöffnet und geschlossen. Das Ventil 6 ist im Einlass-Zeitraum Z1 geöffnet und im Oxidierungs-Zeitraum Z3 geschlossen.
- Die oben beschriebene Lochblende oder auch eine Klappe wird relativ zum Gehäuse 5 bewegt, sodass die Lochblende 5 bzw. die Klappe im Einlass-Zeitraum Z1 den Einlass E freigibt und im Oxidierungs-Zeitraum Z3 den Einlass E versperrt.

Diese beiden Ausgestaltungen lassen sich miteinander kombinieren, was in vielen Fällen die Zuverlässigkeit der Gasdetektionsvorrichtung 100 vergrößert. Im Einlass-Zeitraum Z1 sind gemäß dieser Kombination der Einlass E geöffnet, und das Oxidierungs-Bauteil 2 und das optionale Heizelement 3 sind ausgeschaltet. Im Oxidierungs-Zeitraum Z3 sind der Einlass E geschlossen, das Oxidierungs-Bauteil 2 eingeschaltet und das optionale Heizelement 3 ausgeschaltet. Im optionalen Heiz-Zeitraum Z2 ist bevorzugt der Einlass E geschlossen.

Die nachfolgende Beschreibung bezieht sich auf die Ausgestaltung gemäß Figur 1 und auf beide Ausgestaltungen, also sowohl auf der Ausgestaltung, bei der das Oxidierungs-Bauteil 2 eingeschaltet und ausgeschaltet wird, als auch auf die Ausgestaltung, bei der die Messkammer 9 geöffnet und geschlossen wird.

Die beiden gemessenen Werte r1 und r2 und optional weitere gemessene Werte für den elektrischen Widerstand R werden an eine signalverarbeitende Auswerteeinheit 15 übermittelt, die in der gezeigten Realisierung ein Bestandteil des Steuergeräts 12 ist. Wie bereits dargelegt, ist im Ausführungsbeispiel der elektrische Widerstand R des Halbleiter-Bauteils 10 umso kleiner, je größer der Anteil (die Konzentration) von brennbarem Zielgas in der Messkammer 9 ist. Der elektrische Widerstand R hängt außerdem von Umgebungsbedingungen ab. Erfindungsgemäß wird die Differenz $\Delta$r = r2 - r1 berechnet und ausgewertet, und zwar von der Auswerteeinheit 15. Diese Differenz $\Delta$r hängt im Wesentlichen nur von der gesuchten Konzentration von brennbarem Zielgas in der Messkammer 9 ab, während Umgebungsbedingungen an beiden Mess-Zeitpunkten t1 und t2 etwa gleich auf den elektrischen Widerstand R einwirken. "Im Wesentlichen" bedeutet:

Der Einfluss von Umgebungsbedingungen auf die Differenz $\Delta r$ ist vernachlässigbar gering. Auf diese Weise wird der Einfluss von Umgebungsbedingungen rechnerisch kompensiert.

In einer Ausgestaltung wird die Gasdetektionsvorrichtung 100 für die Entscheidung verwendet, ob in dem zu überwachenden Bereich mindestens ein brennbares Zielgas vorhanden ist oder nicht. Während eines Einsatzes wird wiederholt eine Abfolge Z1, Z2, Z3 durchgeführt. Falls nach einer Abfolge die gemessene Differenz $\Delta r$ oberhalb einer Differenz-Schranke liegt, so ist ein brennbares Zielgas detektiert. Ansonsten steht fest, dass aktuell kein brennbares Zielgas vorhanden ist - natürlich vorausgesetzt, dass die Gasdetektionsvorrichtung 100 intakt ist. Die Erfinder haben in internen Versuchen mit einem bestimmten brennbaren Zielgas festgestellt, dass eine erfindungsgemäße Gasdetektionsvorrichtung 100 dieses Zielgas mit einer Konzentration von unter 10 ppm zuverlässig zu detektieren vermag, oft sogar mit einer Konzentration von unter 2 ppm.

Bevorzugt wird die Gasdetektionsvorrichtung 100 vorab kalibriert. Bei dieser Kalibrierung werden in der Umgebung der Gasdetektionsvorrichtung 100 nacheinander unterschiedliche Konzentrationen con(1), con(2), ... eines zu detektierenden Zielgases hergestellt. Für jede Konzentration con(i) wird jeweils mindestens einmal eine Differenz $\Delta r(i)$ gemessen. Bevorzugt werden mehrere Abfolgen Z1, Z2, Z3 durchgeführt, und über die gemessenen Differenzen wird gemittelt. Die Kalibrierung liefert einen empirisch ermittelten funktionalen Zusammenhang $Con = f(\Delta R)$. Dieser empirisch ermittelte funktionale Zusammenhang wird in einer rechnerauswertbaren Form in einem Datenspeicher der Auswerteeinheit 15 abgespeichert. Anmerkung: Mit Con wird die Größe bezeichnet, mit con ein bestimmter Messwert für diese Größe.

Bevorzugt wird bei dieser Justierung auch das Heizelement 3 so wie oben beschrieben eingestellt, sodass der Eintrag pro Zeiteinheit von Wärmeenergie durch das Heizelement 3 gleich dem Eintrag pro Zeiteinheit durch das Oxidierungs-Bauteil 2 ist.

Bei einem Einsatz der Gasdetektionsvorrichtung 100 wird die Abfolge Z1, Z2, Z3 wiederholt durchgeführt. Auf die gemessene Differenz $\Delta r$ wird der abgespeicherte funktionale Zusammenhang f angewendet und liefert die gesuchte aktuelle Zielgas-Konzentration $con = f(\Delta r)$.

Erfindungsgemäß wird mindestens an den beiden Zeitpunkten t1 und t2 der elektrische Widerstand R des Halbleiter-Bauteils 10 gemessen. In einer Ausgestaltung wird der elektrische Widerstand R zusätzlich an mindestens einem Zwischen-Zeitpunkt t_x, der zwischen den Zeitpunkten t1 und t2 liegt, gemessen. In einer bevorzugten Weiterbildung wird der zeitliche Verlauf des elektrischen Widerstands R im Mess-Zeitraum Z3 gemessen. Durch geeignete numerische Verfahren, beispielsweise mithilfe einer Glättung, werden bis zu einem gewissen Grade Ausreißer und sonstige Messfehler rechnerisch kompensiert.

Bei der zweiten Ausführungsform gemäß Figur 2 umfasst die Gasdetektionsvorrichtung 100 keinen Einlass E und keinen Auslass A. In einer Ausgestaltung vermag eine verschiebbare Blende oder ein sonstiger geeigneter Verschluss die Fluidverbindung zwischen der Messkammer 9 und der Umgebung wahlweise freizugeben oder zu verschließen. Bei dieser Ausgestaltung wird ebenfalls mindestens eine Abfolge mit einem Einlass-Zeitraum Z1, einem Heiz-Zeitraum Z2 und einem Mess-Zeitraum Z3 durchgeführt, wobei die Fluidverbindung zwischen der Messkammer 9 und der Umgebung während des Einlass-Zeitraums Z1 geöffnet und während des Heiz-Zeitraums Z2 und des Mess-Zeitraums Z3 unterbrochen ist. Die Ausgestaltung gemäß Figur 2 und mit dem beweglichen Verschluss lässt sich mit einer Realisierungsform kombinieren, bei denen das Oxidierungs-Bauteil 2 dauerhaft eingeschaltet ist und ein Heizelement 3 zwar vorhanden sein kann, aber nicht benötigt wird.

Falls hingegen dauerhaft eine Fluidverbindung zwischen der Messkammer 9 und der Umgebung durch die Membrane 8 hindurch hergestellt ist, so wird bevorzugt mindestens einmal eine Abfolge durchgeführt, die nur aus einem Heiz-Zeitraum Z2 und einem nachfolgenden Mess-Zeitraum Z3 besteht, wobei das Steuergerät 12 die oben für diese beiden Zeiträume Z2 und Z3 beschriebenen Vorgänge auslöst. Bei dieser Ausgestaltung fungiert der Heiz-Zeitraum Z2 gleichzeitig als der Einlass-Zeitraum Z1.

**Bezugszeichenliste**

| 1 | Halbleiter-Sensor, umfasst das Halbleiter-Bauteil 10 und das Heizelement 11 |
|---|---|
| 2 | ansteuerbares Oxidierungs-Bauteil 2, umfasst den Heizdraht 20, die keramische und katalytische Ummantelung 21, die elektrischen Kontaktierungen 22 und die Platte 23, ist als katalytischer Pellistor ausgestaltet |
| 3 | ansteuerbares Heizelement in der Messkammer 9 |
| 4 | thermische Barriere zwischen dem Oxidierungs-Bauteil 2 und dem Heizelement 3 einerseits und dem Halbleiter-Sensor 1 andererseits |

(fortgesetzt)

| 5 | Gehäuse der Gasdetektionsvorrichtung 100, umgibt die Messkammer 9 |
|---|---|
| 6 | Ventil am Einlass E |
| 7 | Ventil am Auslass A |
| 8 | gasdurchlässige Membrane, verbindet die Messkammer 9 mit der Umgebung |
| 9 | Messkammer der Gasdetektionsvorrichtung 100, vom Gehäuse 5 umgeben, nimmt den Halbleiter-Sensor 1, das Oxidierungs-Bauteil 2, das Heizelement 3 und die thermische Barriere 4 auf |
| 10 | Halbleiter-Bauteil des Halbleiter-Sensors 1, dessen elektrischen Widerstand R gemessen wird, fungiert als das Sensor-Bauteil |
| 11 | Heizelement des Halbleiter-Sensors 1, heizt das Halbleiter-Bauteil 10 |
| 12 | signalverarbeitendes Steuergerät, steuert das Heizelement 11 des Halbleiter-Sensors 1, das Oxidierungs-Bauteil 2, das Heizelement 3, die optionalen Ventile 6 und 7 und die optionale Pumpe 13 an, umfasst die Auswerteeinheit 15 |
| 13 | ansteuerbare Pumpe, welche eine Gasprobe G aus der Umgebung ansaugt und durch den Einlass E hindurch in die Messkammer 9 einspeist |
| 14 | Systemuhr des Steuergeräts 12 |
| 15 | signalverarbeitende Auswerteeinheit, ermittelt die Konzentration des brennbaren Zielgases, ist ein Bestandteil des Steuergeräts 12 |
| 20 | Heizdraht des Oxidierungs-Bauteils 2 |
| 21 | keramische und katalytische Ummantelung des Oxidierungs-Bauteils 2 |
| 22 | elektrische Kontaktierungen des Oxidierungs-Bauteils 2 |
| 23 | Platte des Oxidierungs-Bauteils 2 |
| 24 | Stromstärken-Sensor, misst die Stärke I des Stroms, der durch das Halbleiter-Bauteil 10 fließt |
| 25 | Spannungs-Sensor, misst die elektrische Spannung U, die am Halbleiter-Bauteil 10 anliegt |
| 100 | Gasdetektionsvorrichtung, umfasst die Messkammer 9 im Gehäuse 5, den Halbleiter-Sensor 1, das Oxidierungs-Bauteil 2, das Heizelement 3, die thermische Barriere 4, optional den Einlass E mit dem Ventil 6 und den Auslass A mit dem Ventil 7, optional die Membrane 8 und optional die Pumpe 13 |
| A | Auslass aus der Messkammer 9, umfasst das Ventil 7 |
| dist(2) | Abstand zwischen dem Oxidierungs-Bauteil 2 und dem Halbleiter-Sensor 1 |
| dist(3) | Abstand zwischen dem Heizelement 3 und dem Halbleiter-Sensor 1 |
| E | Einlass zu der Messkammer 9, umfasst das Ventil 6 |
| E1? | Entscheidung: Heiz-Zeitraum Z2 verstrichen? |
| G | Gasprobe aus dem zu überwachenden Bereich, fließt durch den Einlass E oder die Membrane 8 hindurch in die Messkammer 9 und wird dort untersucht |
| R | elektrischer Widerstand des Halbleiter-Bauteils 10, wird aufgrund von Messwerten der Sensoren 24 und 25 ermittelt |
| r1 | Messwert zum Zeitpunkt t1 für den elektrischen Widerstand R |
| r2 | Messwert zum Zeitpunkt t2 für den elektrischen Widerstand R |
| r(t) | Messwert zum Zeitpunkt t für den elektrischen Widerstand R |
| ΔR_min | vorgegebene Schranke für die Veränderung des elektrischen Widerstands R |
| S1 | Schritt: zum Zeitpunkt ta werden die Ventile 6 und 7 geöffnet, die Pumpe 13 wird eingeschaltet, und die Messkammer 9 wird ausgespült |
| S2 | Schritt: zum Zeitpunkt t0 werden die Ventile 6 und 7 geschlossen, und die Pumpe 13 wird eingeschaltet |
| S3 | Schritt: zum Zeitpunkt t0 wird das Heizelement 3 eingeschaltet |

(fortgesetzt)

| | | |
|---|---|---|
| S4 | Schritt: zum Zeitpunkt t1 wird das Heizelement 3 ausgeschaltet, und das Oxidierungs-Bauteil 2 wird eingeschaltet |
| t0 | Anfang des Heiz-Zeitraums Z2 |
| t1 | erster Zeitpunkt, an dem die Detektionsgröße gemessen wird und der als der Detektions-Zeitpunkt fungiert - am ersten Zeitpunkt kann brennbares Zielgas in der Messkammer 9 sein |
| t2 | zweiter Zeitpunkt, an dem die Detektionsgröße gemessen wird und der als der Referenz-Zeitpunkt fungiert - am zweiten Zeitpunkt ist durch das Oxidieren kein brennbares Zielgas in der Messkammer 9 vorhanden |
| ta | Anfang des Einlass-Zeitraums Z1 |
| Z1 | Einlass-Zeitraum, in dem die Gasprobe G in die Messkammer 9 fließt und das Heizelement 3 ausgeschaltet ist |
| Z2 | Heiz-Zeitraum, in dem das Heizelement 3 eingeschaltet und das Oxidierungs-Bauteil 2 ausgeschaltet ist, beginnt im Zeitpunkt t0 |
| Z3 | Oxidierungs-Zeitraum, in dem das Oxidierungs-Bauteil 2 eingeschaltet und das Heizelement 3 ausgeschaltet ist, reicht vom ersten Zeitpunkt t1 bis zum zweiten Zeitpunkt t2 |

**Patentansprüche**

1. Gasdetektionsvorrichtung (100) zum Überwachen eines räumlichen Bereichs auf ein brennbares Zielgas ($CH_4$),

wobei die Gasdetektionsvorrichtung (100)

- eine Messkammer (9),
- ein elektrisch leitendes Sensor-Bauteil (10, 20),
- ein Oxidierungs-Bauteil (2),
- einen Detektions-Sensor (24, 25),
- ein Heizelement (3) und
- eine signalverarbeitende Auswerteeinheit (15)

umfasst,
wobei das Oxidierungs-Bauteil (2) einschaltbar und ausschaltbar ist,
wobei das Heizelement (3) einschaltbar und ausschaltbar ist,
wobei die Gasdetektionsvorrichtung (100) so ausgestaltet ist, dass

- wenigstens zeitweise eine Gasprobe (G) aus dem Bereich in die Messkammer (9) fließt und
- das Sensor-Bauteil (10, 20) in Kontakt mit der Gasprobe (G) in der Messkammer (9) kommt,

wobei das Heizelement (3) dazu ausgestaltet ist, im eingeschalteten Zustand die Gasprobe (G) in der Messkammer (9) zu erhitzen,
wobei das Sensor-Bauteil (10, 20) eine messbare elektrische Detektionsgröße (R), insbesondere einen elektrischen Widerstand, aufweist,
die in einer ersten Realisierungsform umso größer und in einer zweiten Realisierungsform umso kleiner ist, desto geringer die Konzentration des brennbaren Zielgases ($CH_4$) in der Gasprobe (G) in der Messkammer (9) ist,
wobei der Detektions-Sensor (24, 25) dazu ausgestaltet ist, ein Maß für die Detektionsgröße (R) des Sensor-Bauteils (10, 20) zu messen,
wobei das Oxidierungs-Bauteil (2) dazu ausgestaltet ist, brennbares Zielgas ($CH_4$), das in einer Gasprobe (G) in der Messkammer (9) enthalten ist, zu oxidieren,
wobei die Gasdetektionsvorrichtung (100) so ausgestaltet ist, dass
das Oxidierungs-Bauteil (2) in einem Oxidierungs-Zeitraum (Z3) in der Messkammer (9) brennbares Zielgas ($CH_4$) in der Gasprobe (G) vollständig oxidiert,
der Detektions-Sensor (24, 25) sowohl an einem Detektions-Zeitpunkt (t1) als auch an einem Referenz-Zeitpunkt (t2) ein Maß für die Detektionsgröße (R) des Sensor-Bauteils (10, 20) misst,

wobei der Oxidierungs-Zeitraum (Z3) am oder nach dem früheren Zeitpunkt (t1) der beiden Zeitpunkte (t1, t2) beginnt und vor dem oder am späteren Zeitpunkt (t2) der beiden Zeitpunkte (t1, t2) endet, wobei die Gasdetektionsvorrichtung (100) weiterhin so ausgestaltet ist, dass dann, wenn im zu überwachenden Bereich brennbares Zielgas ($CH_4$) vorhanden ist,

- auch am Detektions-Zeitpunkt (t1) in der Messkammer (9) brennbares Zielgas ($CH_4$) vorhanden ist und
- am Referenz-Zeitpunkt (t2) in der Messkammer (9) aufgrund der Oxidation im Oxidierungs-Zeitraum (Z3) kein brennbares Zielgas ($CH_4$) vorhanden ist,

wobei die Auswerteeinheit (15) dazu ausgestaltet ist, die Differenz ($\Delta r$) zwischen dem Messwert (r2) am Referenz-Zeitpunkt (t2) und dem Messwert (r1) am Detektions-Zeitpunkt (t1) für die Detektionsgröße (R) zu berechnen und abhängig von dieser Differenz ($\Delta r$) automatisch die Konzentration des brennbaren Zielgases ($CH_4$) in der Gasprobe (G) zu ermitteln, und wobei die Gasdetektionsvorrichtung (100) so ausgestaltet ist, dass

- in einem Heiz-Zeitraum (Z2) das Heizelement (3) im eingeschalteten Zustand und das Oxidierungs-Bauteil (2) in einem ausgeschalteten Zustand sind und
- im Oxidierungs-Zeitraum (Z3) das Oxidierungs-Bauteil (2) in einem eingeschalteten Zustand und das Heizelement (3) in einem ausgeschalteten Zustand sind.

2. Gasdetektionsvorrichtung (100) nach Anspruch 1,
**dadurch gekennzeichnet, dass**

das Oxidierungs-Bauteil (2) im eingeschalteten Zustand einen Eintrag von Wärmeenergie in das Sensor-Bauteil (10, 20) bewirkt und das Heizelement (3) im eingeschalteten Zustand ebenfalls einen Eintrag von Wärmeenergie in das Sensor-Bauteil (10, 20) bewirkt, wobei der Eintrag von Wärmeenergie pro Zeiteinheit durch das eingeschaltete Heizelement (3) gleich dem Eintrag von Wärmeenergie pro Zeiteinheit durch das eingeschaltete Oxidierungs-Bauteil (2) ist.

3. Gasdetektionsvorrichtung (100) nach Anspruch 1 oder Anspruch 2,
**dadurch gekennzeichnet, dass**

die Gasdetektionsvorrichtung (100) so ausgestaltet ist, dass bei gleichen Umgebungsbedingungen dann, wenn die Gasprobe (G) in der Messkammer (9) kein brennbares Zielgas enthält, die Detektionsgröße (R) des Sensor-Bauteils (10, 20) bei eingeschaltetem Oxidierungs-Bauteil (2) und ausgeschaltetem Heizelement (3) den gleichen Wert annimmt wie bei ausgeschaltetem Oxidierungs-Bauteil (2) und eingeschaltetem Heizelement (3).

4. Gasdetektionsvorrichtung (100) nach einem der vorhergehenden Ansprüche, **dadurch gekennzeichnet, dass**

die Gasdetektionsvorrichtung (100) so ausgestaltet ist, dass der Heiz-Zeitraum (Z2) zeitlich vor dem Oxidierungs-Zeitraum (Z3) liegt. wobei bevorzugt das Ende (t1) des Heiz-Zeitraums (Z2) gleich dem Beginn (t1) des Oxidierungs-Zeitraums (Z3) ist.

5. Gasdetektionsvorrichtung (100) nach einem der vorhergehenden Ansprüche, **dadurch gekennzeichnet, dass**
das Oxidierungs-Bauteil (2)

- im Oxidierungs-Zeitraums (Z3) eingeschaltet und außerhalb des Oxidierungs-Zeitraums (Z3) ausgeschaltet ist.

6. Gasdetektionsvorrichtung (100) nach einem der vorhergehenden Ansprüche, **dadurch gekennzeichnet, dass**

die Gasdetektionsvorrichtung (100) dazu ausgestaltet ist, mindestens einmal eine Steigungs-Berechnungs-Abfolge durchzuführen, wobei die oder jede Steigungs-Berechnungs-Abfolge die Schritte umfasst, dass

- der Detektions-Sensor (24, 25) 25) an mindestens zwei Zeitpunkten (t1, t_x) das Maß für die Detektionsgröße (R) des Sensor-Bauteils (10, 20) misst,

wobei die beiden Zeitpunkte (t1, t_x) im Oxidierungs-Zeitraum (Z3) liegen und zeitlich voneinander beabstandet sind, und

- die Auswerteeinheit (15) abhängig von den oder mindestens zwei Messwerten der Detektionsgröße (R) ein Maß für die Steigung der Detektionsgröße (R) über der Zeit berechnet,

die Auswerteeinheit (15) dazu ausgestaltet ist,
dann, wenn die in einer Steigungs-Berechnungs-Abfolge berechnete Steigung unter einer vorgegebenen Schranke (ΔR_min) liegt,

- den zeitlich jüngsten Zeitpunkt, an dem das Maß für die Detektionsgröße (R) gemessen worden ist, als den Referenz-Zeitpunkt (t2) und
- den Messwert am zeitlich jüngsten Zeitpunkt als den Messwert (r2) am Referenz-Zeitpunkt (t2)

zu verwenden.

7. Gasdetektionsvorrichtung (100) nach einem der vorhergehenden Ansprüche, **dadurch gekennzeichnet, dass**

das Oxidierungs-Bauteil (2)

- im Oxidierungs-Zeitraums (Z3) eingeschaltet und mindestens in einem Einlass-Zeitraum (Z1) ausgeschaltet ist und

die Gasdetektionsvorrichtung (100) so ausgestaltet ist, dass die Gasprobe (G) mindestens im Einlass-Zeitraum (Z1) aus dem räumlichen Bereich in die Messkammer (9) fließt.

8. Gasdetektionsvorrichtung (100) nach Anspruch 7,
**dadurch gekennzeichnet, dass**

die Gasdetektionsvorrichtung (100) so ausgestaltet ist, dass
die Gasprobe (G) auch im Oxidierungs-Zeitraum (Z3), bevorzugt dauerhaft, aus dem räumlichen Bereich in die Messkammer (9) fließt.

9. Gasdetektionsvorrichtung (100) nach einem der vorhergehenden Ansprüche,
**dadurch gekennzeichnet, dass**

die Messkammer (9) wahlweise in einem geöffneten Zustand und in einem geschlossenen Zustand betreibbar ist, wobei die Gasdetektionsvorrichtungen (100) so ausgestaltet ist, dass

- im geöffneten Zustand die Gasprobe (G) in die Messkammer (9) fließt und
- im geschlossenen Zustand die Messkammer (9) gegen den räumlichen Bereich fluiddicht abgedichtet ist, und

wobei die Gasdetektionsvorrichtung (100) so ausgestaltet ist, dass
die Messkammer (9)

- in einem Einlass-Zeitraum (Z1), der den Detektions-Zeitpunkt (t1) umfasst oder vor dem Detektions-Zeitpunkt (t1) liegt, im geöffneten Zustand ist und
- am Referenz-Zeitpunkt (t2) im geschlossenen Zustand ist.

10. Gasdetektionsvorrichtung (100) nach Anspruch 9,
**dadurch gekennzeichnet, dass**

die Gasdetektionsvorrichtung (100) so ausgestaltet ist, dass
das Oxidierungs-Bauteil (2) auch im Einlass-Zeitraum (Z1) eingeschaltet ist und
bevorzugt beim Betrieb der Gasdetektionsvorrichtung (100) dauerhaft eingeschaltet ist.

**11.** Gasdetektionsvorrichtung (100) nach einem der vorhergehenden Ansprüche,
**dadurch gekennzeichnet, dass**
wenigstens dann, wenn das Oxidierungs-Bauteil (2) eingeschaltet ist, elektrischer Strom durch das Sensor-Bauteil (10, 20) fließt.

**12.** Verfahren zum Überwachen eines räumlichen Bereichs auf ein brennbares Zielgas (CH₄)

unter Verwendung einer Gasdetektionsvorrichtung (100), die

- eine Messkammer (9),
- ein elektrisch leitendes Sensor-Bauteil (10, 20),
- einen Detektions-Sensor (24, 25),
- ein Oxidierungs-Bauteil (2) und
- ein Heizelement (3)

umfasst,
wobei das Heizelement (3) sich einschalten und wieder ausschalten lässt, wobei das Oxidierungs-Bauteil (2) sich ebenfalls einschalten und wieder ausschalten lässt und
wobei das Verfahren die Schritte umfasst, dass
ermöglicht oder bewirkt wird, dass wenigstens zeitweise eine Gasprobe (G) aus dem Bereich in die Messkammer (9) fließt,
das Sensor-Bauteil (10, 20) in Kontakt mit der Gasprobe (G) in der Messkammer (9) kommt,
der Kontakt dergestalt einen Einfluss auf das Sensor-Bauteil (10, 20) nimmt, dass eine messbare Detektionsgröße (R), insbesondere der elektrische Widerstand,
des Sensor-Bauteils (10, 20) in einer ersten Realisierungsform umso größer und in einer zweiten Realisierungsform umso kleiner ist, desto geringer die Konzentration des brennbaren Zielgases (CH₄) in der Gasprobe (G) in der Messkammer (9) ist,
das Oxidierungs-Bauteil (2) in einem Oxidierungs-Zeitraum (Z3) brennbares Zielgas (CH₄), das in einer Gasprobe (G) in der Messkammer (9) enthalten ist, vollständig oxidiert,
der Detektions-Sensor (24, 25) sowohl an einem Detektions-Zeitpunkt (t1) als auch an einem Referenz-Zeitpunkt (t2) ein Maß für die Detektionsgröße (R) des Sensor-Bauteils (10, 20) misst,
wobei der Oxidierungs-Zeitraum (Z3) am oder nach dem früheren Zeitpunkt (t1) der beiden Zeitpunkte (t1, t2) beginnt und vor dem oder am späteren Zeitpunkt (t2) der beiden Zeitpunkte (t1, t2) endet,
wobei dann, wenn im zu überwachenden Bereich brennbares Zielgas (CH₄) vorhanden ist,

- auch am Detektions-Zeitpunkt (t1) in der Messkammer (9) brennbares Zielgas (CH₄) vorhanden ist und
- am Referenz-Zeitpunkt (t2) in der Messkammer (9) aufgrund der Oxidation im Oxidierungs-Zeitraum (Z3) kein brennbares Zielgas (CH₄), vorhanden ist,

die Differenz (Δr) zwischen dem Messwert (r2) am Referenz-Zeitpunkt (t2) und dem Messwert (r1) am Detektions-Zeitpunkt (t1) berechnet wird und
abhängig von dieser Differenz (Δr) automatisch die Konzentration des brennbaren Zielgases (CH₄) in der Gasprobe (G) ermittelt wird und
das Verfahren die weiteren Schritte umfasst, dass

- das Oxidierungs-Bauteil (2) zu Beginn des Oxidierungs-Zeitraums (Z3) eingeschaltet und am Ende des Oxidierungs-Zeitraums (Z3) ausgeschaltet wird,
- das Heizelement (3) zu Beginn eines Heiz-Zeitraums (Z2), welcher außerhalb des Oxidierungs-Zeitraums (Z3) liegt, eingeschaltet und am Ende des Heiz-Zeitraums (Z2) wieder ausgeschaltet wird und
- das eingeschaltete Heizelement (3) die Gasprobe (G) in der Messkammer (9) erhitzt.

**13.** Verfahren nach Anspruch 12,
**dadurch gekennzeichnet, dass**
der Wärmeeintrag pro Zeiteinheit des eingeschalteten Heizelements (3) in das Sensor-Bauteil (10, 20) gleich dem Wärmeeintrag pro Zeiteinheit des eingeschalteten Oxidierungs-Bauteils (2) in das Sensor-Bauteil (10, 20) ist.

**14.** Verfahren nach Anspruch 12 oder Anspruch 13,
**dadurch gekennzeichnet, dass**

mindestens einmal eine Steigungs-Berechnungs-Abfolge durchgeführt wird,
wobei die oder jede Steigungs-Berechnungs-Abfolge die Schritte umfasst, dass

- der Detektions-Sensor (24, 25) 25) an mindestens zwei Zeitpunkten (t1, t_x) das Maß für die Detektionsgröße (R) des Sensor-Bauteils (10, 20) misst,
wobei die beiden Zeitpunkte (t1, t_x) im Oxidierungs-Zeitraum (Z3) liegen und zeitlich voneinander beabstandet sind, und
- abhängig von den oder mindestens zwei Messwerten der Detektionsgröße (R) ein Maß für die Steigung der Detektionsgröße (R) über der Zeit berechnet wird und

dann, wenn die berechnete Steigung unter einer vorgegebenen Schranke (ΔR_min) liegt,

- der zeitlich jüngste Zeitpunkt, an dem das Maß für die Detektionsgröße (R) gemessen worden ist, als der Referenz-Zeitpunkt (t2) und
- der Messwert am zeitlich jüngsten Zeitpunkt als der Messwert (r2) am Referenz-Zeitpunkt (t2)

verwendet werden.

15. Verfahren nach einem der Ansprüche 12 bis 14,
**dadurch gekennzeichnet, dass**

eine erste Abfolge und / oder eine zweite Abfolge durchgeführt werden,
wobei die erste Abfolge die Schritte umfasst, dass

- das Oxidierungs-Bauteil (2) in einem Einlass-Zeitraum (Z1), der bevorzugt den Detektions-Zeitpunkt (t1) umfasst oder vor dem Detektions-Zeitpunkt (t1) liegt, ausgeschaltet ist und
- die Gasprobe (G) mindestens im Einlass-Zeitraum (Z1) in die Messkammer (9) fließt und

wobei die zweite Abfolge die Schritte umfasst, dass

- die Messkammer (9) im Einlass-Zeitraum (Z1) in einem geöffneten Zustand betrieben wird, in dem die Gasprobe (G) aus dem zu überwachenden räumlichen Bereich in die Messkammer (9) fließt, und
- die Messkammer (9) in einem geschlossenen Zustand betrieben wird, in dem die Messkammer (9) gegen den Bereich fluiddicht abgedichtet ist,
wobei bevorzugt die Messkammer (9) zum Detektions-Zeitpunkt (t1) im geöffneten Zustand betrieben oder vom geschlossenen in den geöffneten Zustand überführt wird, und
- das Oxidierungs-Bauteil (2) wenigstens dann eingeschaltet ist, wenn die Messkammer (9) im geschlossenen Zustand betrieben wird.

## Claims

1. Gas detection device (100) for monitoring a spatial region for a combustible target gas ($CH_4$),

wherein the gas detection device (100) comprises

- a measuring chamber (9),
- an electrically conductive sensor component (10, 20),
- an oxidation component (2),
- a detection sensor (24, 25),
- a heating element (3) and
- a signal-processing evaluation unit (15),

wherein the oxidation component (2) can be switched on and off,
wherein the heating element (3) can be switched on and off,
wherein the gas detection device (100) is configured such that

- a gas sample (G) flows at least temporarily from the region into the measuring chamber (9) and

- the sensor component (10, 20) comes into contact with the gas sample (G) in the measuring chamber (9),

wherein the heating element (3) is configured to heat the gas sample (G) in the measuring chamber (9) in the switched-on state,
wherein the sensor component (10, 20) has a measurable electrical detection variable (R), in particular an electrical resistance,
which is greater in a first embodiment and smaller in a second embodiment, the lower the concentration of the combustible target gas ($CH_4$) in the gas sample (G) in the measuring chamber (9), wherein the detection sensor (24, 25) is configured to measure a measurement of the detection variable (R) of the sensor component (10, 20),
wherein the oxidation component (2) is configured to oxidize combustible target gas ($CH_4$) contained in a gas sample (G) in the measuring chamber (9),
wherein the gas detection device (100) is configured such that
the oxidation component (2) completely oxidizes combustible target gas ($CH_4$) in the gas sample (G) in the measuring chamber (9) in an oxidation period (Z3),
the detection sensor (24, 25) measures a measurement of the detection variable (R) of the sensor component (10, 20) both at a detection time (t1) and at a reference time (t2),
wherein the oxidation period (Z3) begins at or after the earlier time (t1) of the two times (t1, t2) and ends before or at the later time (t2) of the two times (t1, t2),
wherein the gas detection device (100) is furthermore configured such that
when combustible target gas ($CH_4$) is present in the region to be monitored,

- combustible target gas ($CH_4$) is also present in the measuring chamber (9) at the detection time (t1) and
- combustible target gas ($CH_4$) is not present in the measuring chamber (9) at the reference time (t2) in the oxidation period (Z3) due to the oxidation,

wherein the evaluation unit (15) is configured to
calculate the difference ($\Delta r$) between the measured value (r2) at the reference time (t2) and the measured value (r1) at the detection time (t1) for the detection variable (R) and
depending on this difference ($\Delta r$), automatically determine the concentration of the combustible target gas ($CH_4$) in the gas sample (G), and
wherein the gas detection device (100) is configured such that

- in a heating period (Z2) the heating element (3) is in the switched-on state and the oxidation component (2) is in a switched-off state and
- in the oxidation period (Z3) the oxidation component (2) is in a switched-on state and the heating element (3) is in a switched-off state.

2. Gas detection device (100) according to claim 1,
   **characterized in that**

   in the switched-on state, the oxidation component (2) causes heat energy to be introduced into the sensor component (10, 20) and
   the heating element (3) also causes an input of heat energy into the sensor component (10, 20) in the switched-on state,
   the input of heat energy per time unit by the switched-on heating element (3) being equal to the input of heat energy per time unit by the switched-on oxidation component (2).

3. Gas detection device (100) according to either claim 1 or claim 2,
   **characterized in that**

   the gas detection device (100) is configured such that, under the same ambient conditions, if the gas sample (G) in the measuring chamber (9) does not contain any combustible target gas,
   the detection variable (R) of the sensor component (10, 20) assumes the same value when the oxidation component (2) is switched on and the heating element (3) is switched off as when the oxidation component (2) is switched off and the heating element (3) is switched on.

4. Gas detection device (100) according to any of the preceding claims, **characterized in that**
   the gas detection device (100) is configured such that

the heating period (Z2) is temporally before the oxidation period (Z3), preferably the end (t1) of the heating period (Z2) being equal to the beginning (t1) of the oxidation period (Z3).

5. Gas detection device (100) according to any of the preceding claims, **characterized in that** the oxidation component (2)

- is switched on during the oxidation period (Z3) and switched off outside the oxidation period (Z3).

6. Gas detection device (100) according to any of the preceding claims, **characterized in that**

the gas detection device (100) is configured to carry out a gradient calculation sequence at least once, the or each gradient calculation sequence comprising the steps whereby:

- the detection sensor (24, 25) 25) measures the measurement of the detection variable (R) of the sensor component (10, 20) at at least two times (t1, t_x),

the two times (t1, t_x) being in the oxidation period (Z3) and being spaced apart in time, and

- the evaluation unit (15) calculates a measurement of the gradient of the detection variable (R) over time depending on the one or at least two measured values of the detection variable (R),

the evaluation unit (15) is configured to, when the gradient calculated in a gradient calculation sequence is below a given limit ($\Delta R\_min$),

- use the most recent time at which the measurement of the detection variable (R) was measured as the reference time (t2) and
- use the measured value at the most recent time as the measured value (r2) at the reference time (t2).

7. Gas detection device (100) according to any of the preceding claims, **characterized in that**

the oxidation component (2)

- is switched on during the oxidation period (Z3) and switched off during at least one inlet period (Z1) and

the gas detection device (100) is configured such that the gas sample (G) flows from the spatial region into the measuring chamber (9) at least during the inlet period (Z1).

8. Gas detection device (100) according to claim 7,
**characterized in that**

the gas detection device (100) is configured such that
the gas sample (G) flows from the spatial region into the measuring chamber (9) even during the oxidation period (Z3), preferably permanently.

9. Gas detection device (100) according to any of the preceding claims, **characterized in that**

the measuring chamber (9) can be operated selectively in an open state and in a closed state, the gas detection devices (100) being configured such that

- in the open state, the gas sample (G) flows into the measuring chamber (9) and
- in the closed state, the measuring chamber (9) is sealed so as to be fluid-tight against the spatial region, and

the gas detection device (100) being configured such that
the measuring chamber (9)

- is in the open state in an inlet period (Z1) which includes the detection time (t1) or is before the detection time (t1) and
- is in the closed state at the reference time (t2).

**10.** Gas detection device (100) according to claim 9,
**characterized in that**

the gas detection device (100) is configured such that
the oxidation component (2) is also switched on during the inlet period (Z1) and
preferably permanently switched on during operation of the gas detection device (100).

**11.** Gas detection device (100) according to any of the preceding claims,
**characterized in that**
at least when the oxidation component (2) is switched on, electrical current flows through the sensor component (10, 20).

**12.** Method for monitoring a spatial region for a combustible target gas ($CH_4$) using a gas detection device (100) which comprises

- a measuring chamber (9),
- an electrically conductive sensor component (10, 20),
- a detection sensor (24, 25),
- an oxidation component (2) and
- a heating element (3),

wherein the heating element (3) can be switched on and off again, wherein the oxidation component (2) can also be switched on and off again and
wherein the method comprises the following steps whereby:

it is made possible or effected that a gas sample (G) flows at least temporarily from the region into the measuring chamber (9),
the sensor component (10, 20) comes into contact with the gas sample (G) in the measuring chamber (9),
the contact influences the sensor component (10, 20) in such a way that a measurable detection variable (R), in particular the electrical resistance of the sensor component (10, 20), is greater in a first embodiment and smaller in a second embodiment, the lower the concentration of the combustible target gas ($CH_4$) in the gas sample (G) in the measuring chamber (9),
the oxidation component (2) completely oxidizes combustible target gas ($CH_4$) contained in a gas sample (G) in the measuring chamber (9) in an oxidation period (Z3),
the detection sensor (24, 25) measures a measurement of the detection variable (R) of the sensor component (10, 20) both at a detection time (t1) and at a reference time (t2),
wherein the oxidation period (Z3) begins at or after the earlier time (t1) of the two times (t1, t2) and ends before or at the later time (t2) of the two times (t1, t2),
wherein, if combustible target gas ($CH_4$) is present in the region to be monitored,

- combustible target gas ($CH_4$) is also present in the measuring chamber (9) at the detection time (t1) and
- combustible target gas ($CH_4$) is not present in the measuring chamber (9) at the reference time (t2) in the oxidation period (Z2) due to the oxidation,

the difference ($\Delta r$) between the measured value (r2) at the reference time (t2) and the measured value (r1) at the detection time (t1) is calculated and
depending on this difference ($\Delta r$), the concentration of the combustible target gas ($CH_4$) in the gas sample (G) is automatically determined and
the method comprises the further steps whereby:

- the oxidation component (2) is switched on at the beginning of the oxidation period (Z3) and switched off at the end of the oxidation period (Z3),
- the heating element (3) is switched on at the beginning of a heating period (Z2) which is outside the oxidation period (Z3) and switched off again at the end of the heating period (Z2) and
- the switched-on heating element (3) heats the gas sample (G) in the measuring chamber (9).

**13.** Method according to claim 12,
**characterized in that**

the heat input per time unit of the switched-on heating element (3) into the sensor component (10, 20) is equal to the heat input per time unit of the switched-on oxidation component (2) into the sensor component (10, 20).

14. Method according to either claim 12 or claim 13,
    **characterized in that**

    a gradient calculation sequence is carried out at least once,
    the or each gradient calculation sequence comprising the steps whereby:

        - the detection sensor (24, 25) 25) measures the measurement of the detection variable (R) of the sensor component (10, 20) at at least two times (t1, t_x),

    the two times (t1, t_x) being in the oxidation period (Z3) and being spaced apart in time, and

        - depending on the one or at least two measured values of the detection variable (R), a measurement of the gradient of the detection variable (R) over time is calculated and

    when the calculated gradient is below a given limit (△R_min),

        - the most recent time at which the measurement of the detection variable (R) was measured is used as the reference time (t2) and
        - the measured value at the most recent time is used as the measured value (r2) at the reference time (t2).

15. Method according to any of claims 12 to 14,
    **characterized in that**

    a first sequence and/or a second sequence are carried out,
    the first sequence comprising the steps whereby:

        - the oxidation component (2) is switched off in an inlet period (Z1), which preferably includes the detection time (t1) or is before the detection time (t1), and
        - the gas sample (G) flows into the measuring chamber (9) at least during the inlet period (Z1) and

    the second sequence comprising the steps whereby

        - the measuring chamber (9) is operated in an open state during the inlet period (Z1), in which the gas sample (G) flows from the spatial region to be monitored into the measuring chamber (9), and
        - the measuring chamber (9) is operated in a closed state in which the measuring chamber (9) is sealed so as to be fluid-tight against the region,

    the measuring chamber (9) being preferably operated in the open state at the detection time (t1) or being transferred from the closed to the open state, and

        - the oxidation component (2) being switched on at least when the measuring chamber (9) is operated in the closed state.

**Revendications**

1. Dispositif de détection de gaz (100) pour la surveillance d'une zone spatiale pour un gaz cible combustible (CH$_4$),

    dans lequel le dispositif de détection de gaz (100) comprend

        - une chambre de mesure (9),
        - un composant formant capteur (10, 20) électriquement conducteur,
        - un composant d'oxydation (2),
        - un capteur de détection (24, 25),
        - un élément chauffant (3) et

- une unité d'évaluation (15) traitant des signaux,

dans lequel le composant d'oxydation (2) peut être activé et désactivé,
dans lequel l'élément chauffant (3) peut être activé et désactivé,
dans lequel le dispositif de détection de gaz (100) est configuré de sorte que

- un échantillon de gaz (G) s'écoule au moins temporairement de la zone dans la chambre de mesure (9) et
- le composant formant capteur (10, 20) entre en contact avec l'échantillon de gaz (G) dans la chambre de mesure (9),

dans lequel l'élément chauffant (3) est conçu pour chauffer l'échantillon de gaz (G) dans la chambre de mesure (9) lorsqu'il est activé,
dans lequel le composant formant capteur (10, 20) présente une grandeur de détection (R) électrique mesurable, en particulier une résistance électrique,
qui est d'autant plus grande dans un premier mode de réalisation et d'autant plus petite dans un second mode de réalisation que la concentration du gaz cible combustible ($CH_4$) dans l'échantillon de gaz (G) dans la chambre de mesure (9) est faible, dans lequel le capteur de détection (24, 25) est configuré pour mesurer une mesure pour la grandeur de détection (R) du composant formant capteur (10, 20),
dans lequel le composant d'oxydation (2) est conçu pour oxyder un gaz cible combustible ($CH_4$) contenu dans un échantillon de gaz (G) dans la chambre de mesure (9),
dans lequel le dispositif de détection de gaz (100) est configuré de sorte que
le composant d'oxydation (2) oxyde complètement le gaz cible combustible ($CH_4$) dans l'échantillon de gaz (G) pendant une période d'oxydation (Z3) dans la chambre de mesure (9),
le capteur de détection (24, 25) mesure une mesure pour la grandeur de détection (R) du composant formant capteur (10, 20) à la fois à un instant de détection (t1) et à un instant de référence (t2),
dans lequel la période d'oxydation (Z3) commence à l'instant le plus précoce (t1) parmi les deux instants (t1, t2) ou après celui-ci et se termine avant l'instant le plus tardif (t2) parmi les deux instants (t1, t2) ou à celui-ci,
dans lequel le dispositif de détection de gaz (100) est en outre configuré de sorte que
lorsqu'un gaz cible combustible ($CH_4$) est présent dans la zone à surveiller,

- un gaz cible combustible ($CH_4$) est également présent dans la chambre de mesure (9) à l'instant de détection (t1) et
- à l'instant de référence (t2), un gaz cible combustible ($CH_4$) n'est pas présent dans la chambre de mesure (9) en raison de l'oxydation pendant la période d'oxydation (Z3),

dans lequel l'unité d'évaluation (15) est configurée pour
calculer la différence ($\Delta r$) entre la valeur de mesure (r2) à l'instant de référence (t2) et la valeur de mesure (r1) à l'instant de détection (t1) pour la grandeur de détection (R) et
en fonction de ladite différence ($\Delta r$), déterminer automatiquement la concentration du gaz combustible cible ($CH_4$) dans l'échantillon de gaz (G), et
dans lequel le dispositif de détection de gaz (100) est configuré de sorte que

- pendant une période de chauffage (Z2), l'élément chauffant (3) est à l'état activé et le composant d'oxydation (2) est à l'état désactivé et
- pendant la période d'oxydation (Z3), le composant d'oxydation (2) est dans un état activé et l'élément chauffant (3) est dans un état désactivé.

**2.** Dispositif de détection de gaz (100) selon la revendication 1,
**caractérisé en ce que**

le composant d'oxydation (2), dans l'état activé, provoque un apport d'énergie thermique dans le composant formant capteur (10, 20) et
l'élément chauffant (3), dans l'état activé, provoque également un apport d'énergie thermique dans le composant formant capteur (10, 20),
dans lequel l'apport d'énergie thermique par unité de temps par l'élément chauffant (3) activé est égal à l'apport d'énergie thermique par unité de temps par le composant d'oxydation (2) activé.

**3.** Dispositif de détection de gaz (100) selon la revendication 1 ou la revendication 2,

**caractérisé en ce que**

le dispositif de détection de gaz (100) est configuré de sorte que, dans des conditions ambiantes identiques, lorsque l'échantillon de gaz (G) dans la chambre de mesure (9) ne contient pas de gaz cible combustible, la grandeur de détection (R) du composant formant capteur (10, 20) prend la même valeur lorsque le composant d'oxydation (2) est activé et l'élément chauffant (3) est désactivé que lorsque le composant d'oxydation (2) est désactivé et l'élément chauffant (3) est activé.

4. Dispositif de détection de gaz (100) selon l'une des revendications précédentes, **caractérisé en ce que**

le dispositif de détection de gaz (100) est configuré de sorte que
la période de chauffage (Z2) se situe dans le temps avant la période d'oxydation (Z3), dans lequel, de préférence, la fin (t1) de la période de chauffage (Z2) est égale au début (t1) de la période d'oxydation (Z3).

5. Dispositif de détection de gaz (100) selon l'une des revendications précédentes, **caractérisé en ce que**
le composant d'oxydation (2)

- est activé pendant la période d'oxydation (Z3) et désactivé en dehors de la période d'oxydation (Z3).

6. Dispositif de détection de gaz (100) selon l'une des revendications précédentes, **caractérisé en ce que**

le dispositif de détection de gaz (100) est configuré pour exécuter au moins une fois une séquence de calcul d'augmentation,
dans lequel la ou chaque séquence de calcul d'augmentation comprend les étapes selon lesquelles

- le capteur de détection (24, 25) 25) mesure à au moins deux instants (t1, t_x) la mesure pour la grandeur de détection (R) du composant formant capteur (10, 20),

dans lequel les deux instants (t1, t_x) sont situés dans la période d'oxydation (Z3) et sont espacés dans le temps l'un de l'autre, et

- l'unité d'évaluation (15) calcule, en fonction des ou d'au moins deux valeurs de mesure de la grandeur de détection (R), une mesure pour l'augmentation de la grandeur de détection (R) en fonction du temps,

l'unité d'évaluation (15) est configurée pour
lorsque l'augmentation calculée dans une séquence de calcul d'augmentation est inférieure à une limite prédéfinie ($\Delta$R_min),

- utiliser l'instant le plus récent dans le temps auquel la mesure pour la grandeur de détection (R) a été mesurée comme instant de référence (t2) et
- utiliser la valeur de mesure à l'instant le plus récent dans le temps comme valeur de mesure (r2) à l'instant de référence (t2).

7. Dispositif de détection de gaz (100) selon l'une des revendications précédentes, **caractérisé en ce que**

le composant d'oxydation (2)

- est activé pendant la période d'oxydation (Z3) et désactivé pendant au moins une période d'admission (Z1) et

le dispositif de détection de gaz (100) est conçu de sorte que l'échantillon de gaz (G) s'écoule hors de la zone spatiale dans la chambre de mesure (9) au moins pendant la période d'admission (Z1).

8. Dispositif de détection de gaz (100) selon la revendication 7,
**caractérisé en ce que**

le dispositif de détection de gaz (100) est configuré de sorte que
l'échantillon de gaz (G) s'écoule également pendant la période d'oxydation (Z3), de préférence de manière permanente, hors de la zone spatiale dans la chambre de mesure (9).

9. Dispositif de détection de gaz (100) selon l'une des revendications précédentes, **caractérisé en ce que**

la chambre de mesure (9) peut fonctionner au choix dans un état ouvert et dans un état fermé,
dans lequel le dispositif de détection de gaz (100) est configuré de sorte que

- dans l'état ouvert, l'échantillon de gaz (G) s'écoule dans la chambre de mesure (9) et
- dans l'état fermé, la chambre de mesure (9) est rendue étanche aux fluides par rapport à la zone spatiale, et

dans lequel le dispositif de détection de gaz (100) est configuré de sorte que
la chambre de mesure (9)

- est dans l'état ouvert pendant une période d'admission (Z1) qui comprend l'instant de détection (t1) ou est antérieure à l'instant de détection (t1) et
- est dans l'état fermé à l'instant de référence (t2).

10. Dispositif de détection de gaz (100) selon la revendication 9,
**caractérisé en ce que**

le dispositif de détection de gaz (100) est configuré de sorte que
le composant d'oxydation (2) est également activé pendant la période d'admission (Z1) et
de préférence, est activé en permanence lors du fonctionnement du dispositif de détection de gaz (100).

11. Dispositif de détection de gaz (100) selon l'une des revendications précédentes,
**caractérisé en ce que**
au moins lorsque le composant d'oxydation (2) est activé, un courant électrique circule à travers le composant formant capteur (10, 20).

12. Procédé pour la surveillance d'une zone spatiale pour un gaz cible combustible ($CH_4$) à l'aide d'un dispositif de détection de gaz (100) qui comprend

- une chambre de mesure (9),
- un composant formant capteur (10, 20) électriquement conducteur,
- un capteur de détection (24, 25),
- un composant d'oxydation (2) et
- un élément chauffant (3),

dans lequel l'élément chauffant (3) peut être activé et désactivé, dans lequel le composant d'oxydation (2) peut également être activé et désactivé et
dans lequel le procédé comprend les étapes selon lesquelles
on permet ou provoque l'écoulement d'un échantillon de gaz (G) au moins temporairement depuis la zone dans la chambre de mesure (9),
le composant formant capteur (10, 20) entre en contact avec l'échantillon de gaz (G) dans la chambre de mesure (9),
le contact exerce une influence sur le composant formant capteur (10, 20) de telle sorte qu'une grandeur de détection (R) mesurable, en particulier la résistance électrique, du composant formant capteur (10, 20) est d'autant plus grande dans un premier mode de réalisation et d'autant plus petite dans un second mode de réalisation que la concentration du gaz cible combustible ($CH_4$) dans l'échantillon de gaz (G) dans la chambre de mesure (9) est faible,
le composant d'oxydation (2) oxyde complètement, dans une période d'oxydation (Z3), un gaz cible combustible ($CH_4$) contenu dans un échantillon de gaz (G) dans la chambre de mesure (9),
le capteur de détection (24, 25) mesure une mesure pour la grandeur de détection (R) du composant formant capteur (10, 20) à la fois à un instant de détection (t1) et à un instant de référence (t2),
dans lequel la période d'oxydation (Z3) commence à l'instant le plus précoce (t1) parmi les deux instants (t1, t2) ou après celui-ci et se termine avant l'instant le plus tardif (t2) parmi les deux instants (t1, t2) ou à celui-ci,
dans lequel, si un gaz cible combustible ($CH_4$) est présent dans la zone à surveiller,

- un gaz cible combustible ($CH_4$) est également présent dans la chambre de mesure (9) à l'instant de détection (t1) et

- à l'instant de référence (t2), un gaz cible combustible ($CH_4$) n'est pas présent dans la chambre de mesure (9) en raison de l'oxydation pendant la période d'oxydation (Z3),

la différence ($\Delta r$) entre la valeur de mesure (r2) à l'instant de référence (t2) et la valeur de mesure (r1) à l'instant de détection (t1) est calculée et

en fonction de ladite différence ($\Delta r$), la concentration du gaz combustible cible ($CH_4$) dans l'échantillon de gaz (G) est automatiquement déterminée et

le procédé comprend les étapes supplémentaires selon lesquelles

- le composant d'oxydation (2) est activé au début de la période d'oxydation (Z3) et est désactivé à la fin de la période d'oxydation (Z3),

- l'élément chauffant (3) est activé au début d'une période de chauffage (Z2) qui se situe en dehors de la période d'oxydation (Z3) et est désactivé à la fin de la période de chauffage (Z2) et

- l'élément chauffant (3) activé chauffe l'échantillon de gaz (G) dans la chambre de mesure (9).

13. Procédé selon la revendication 12,
**caractérisé en ce que**
l'apport de chaleur par unité de temps de l'élément chauffant (3) activé dans le composant formant capteur (10, 20) est égal à l'apport de chaleur par unité de temps du composant d'oxydation (2) activé dans le composant formant capteur (10, 20).

14. Procédé selon la revendication 12 ou la revendication 13,
**caractérisé en ce que**

une séquence de calcul d'augmentation est exécutée au moins une fois,
dans lequel la ou chaque séquence de calcul d'augmentation comprend les étapes selon lesquelles

- le capteur de détection (24, 25) 25) mesure à au moins deux instants (t1, t_x) la mesure pour la grandeur de détection (R) du composant formant capteur (10, 20),

dans lequel les deux instants (t1, t_x) sont situés dans la période d'oxydation (Z3) et sont espacés dans le temps l'un de l'autre, et

- en fonction des ou d'au moins deux valeurs de mesure de la grandeur de détection (R), une mesure pour l'augmentation de la grandeur de détection (R) en fonction du temps est calculée et

lorsque l'augmentation calculée est inférieure à une limite prédéfinie ($\Delta R\_min$),

- l'instant le plus récent dans le temps auquel la mesure pour la grandeur de détection (R) a été mesurée est utilisé comme instant de référence (t2), et

- la valeur de mesure à l'instant le plus récent dans le temps est utilisée comme valeur de mesure (r2) à l'instant de référence (t2).

15. Procédé selon l'une des revendications 12 à 14,
**caractérisé en ce que**

une première séquence et/ou une seconde séquence sont exécutées,
dans lequel la première séquence comprend les étapes selon lesquelles

- le composant d'oxydation (2) est désactivé pendant une période d'admission (Z1) qui comprend de préférence l'instant de détection (t1) ou est antérieure à l'instant de détection (t1) et

- l'échantillon de gaz (G) s'écoule dans la chambre de mesure (9) au moins pendant la période d'admission (Z1) et

dans lequel la seconde séquence comprend les étapes selon lesquelles

- la chambre de mesure (9) fonctionne pendant la période d'admission (Z1) dans un état ouvert dans lequel l'échantillon de gaz (G) s'écoule hors de la zone spatiale à surveiller dans la chambre de mesure (9) et
- la chambre de mesure (9) fonctionne dans un état fermé dans lequel la chambre de mesure (9) est rendue étanche aux fluides par rapport à la zone,

dans lequel, de préférence, la chambre de mesure (9) fonctionne dans l'état ouvert à l'instant de détection (t1) ou est transférée de l'état fermé à l'état ouvert, et

- le composant d'oxydation (2) est activé au moins lorsque la chambre de mesure (9) fonctionne dans l'état fermé.

FIG. 1

FIG. 2

FIG. 3

EP 4 266 034 B1

**FIG. 4**

FIG. 5

START

ta:S1

t0:S2

t0:S3

E1?

Nein

Ja

①

①

t1:S4

t := t1

S5(t)

r1

③

t := t+Δt

S5(t)

②

②

r(t) − r(t-Δt) < ΔR_min?

Nein

③

Ja

t2 := t

r2

Δr = r2-r1

t2:S6

ENDE

EP 4 266 034 B1

**IN DER BESCHREIBUNG AUFGEFÜHRTE DOKUMENTE**

*Diese Liste der vom Anmelder aufgeführten Dokumente wurde ausschließlich zur Information des Lesers aufgenommen und ist nicht Bestandteil des europäischen Patentdokumentes. Sie wurde mit größter Sorgfalt zusammengestellt; das EPA übernimmt jedoch keinerlei Haftung für etwaige Fehler oder Auslassungen.*

**In der Beschreibung aufgeführte Patentdokumente**

- DE 102008028682 A1